# EUROPEAN PATENT APPLICATION

(11) **EP 3 879 272 A1**
(43) Date of publication of application: **15.09.2021**
(21) Application number: 20161869.1
(22) Date of filing: 09.03.2020
(51) Int. Cl.: G01N 33/68

(54) **GUT MICROBIOTA-RELATED METHODS FOR TREATING DEMENTIA AND AGE-DEPENDENT COGNITIVE DECLINE**

(71) Applicant: Albert-Ludwigs-Universität Freiburg, 79098 Freiburg (DE)
(72) Inventor: Dr. Blank, Thomas, 79199 Kirchzarten (DE)
(74) Representative: Peterreins Schley

(57) **Abstract**

The present invention concerns new approaches for the diagnosis and treatment of dementia diseases. In particular, the present invention pertains to new markers for diagnosing dementia diseases as well as to new targets for the treatment of dementia diseases.

## Description

### Technical Field

The present invention concerns new approaches for the diagnosis and treatment of dementia diseases. In particular, the present invention pertains to new markers for diagnosing dementia diseases as well as to new targets for the treatment of dementia diseases.

### Background

Dementia is the loss of cognitive functioning - thinking, remembering, and reasoning - and behavioral abilities to such an extent that it interferes with a person's daily life and activities. These functions include memory, language skills, visual perception, problem solving, self-management, and the ability to focus and pay attention. Some people with dementia cannot control their emotions, and their personalities may change. Dementia ranges in severity from the mildest stage, when it is just beginning to affect a person's functioning, to the most severe stage, when the person must depend completely on others for basic activities of living.

Signs and symptoms of dementia can result when once-healthy neurons (nerve cells) in the brain stop working, lose connections with other brain cells, and die. While everyone loses some neurons as they age, people with dementia typically experience far greater loss. While dementia is more common as people grow older (up to half of all people age 85 or older may have some form of dementia), it is not a normal part of aging. Many people live into their 90s and beyond without any signs of dementia. One type of dementia, frontotemporal disorders, is more common in middle-aged than older adults. The causes of dementia can vary, depending on the types of brain changes that may be taking place. Alzheimer's disease is the most common cause of dementia in older adults. Other dementias include Lewy body dementia, frontotemporal disorders, and vascular dementia. It is common for people to have mixed dementia - a combination of two or more types of dementia. For example, some people have both Alzheimer's disease and vascular dementia.

In general, dementia is dramatically increasing in importance in society due to demographic change. So far, therapy options have been limited. In recent years there has been an enormous increase in knowledge about the causes and pathophysiology of dementia. Nevertheless, the question of how to diagnose and treat dementia poses a great challenge. An increasing number of research studies demonstrates the enormous importance of the "gut-brain" axis and suggest that triggers for a variety of neurological diseases can be found in the gastrointestinal tract. Research studies have increasingly put emphasis on the influence of microorganisms on host behavior and its cognitive function. Experiments on germ-free animal models show the appearance of behavioral disorders and reduced cognitive functions. Several other animal and human patients studies confirmed that various pathological behaviors as seen in anxiety-like behavior, depression, attention deficiency disorders and cognitive impairments are modulated by gut metabolites (Bercik et al., 2011; Caspani et al., 2019; Dam et al., 2019). A well-studied example of the effect of dysbiosis on cognitive function is liver encephalopathy (that can lead to dementia), which is somewhat positively affected by oral antibiotic therapy (Ahluwalia et al., 2016). Multivariable analyses adjusted for traditional risk factors revealed that a lower prevalence of *Bacteroides* and a higher prevalence of other bacteria are independently and strongly associated with dementia (Saji et al., 2019). A 2015 case report described a 57-year-old male with cirrhosis secondary to both alcohol and the hepatitis C (HCV) virus, decompensated by grade 2 portal systemic encephalopathy. Via a universal stool donor, this patient underwent fecal microbiota transplantation (FMT) with a reduction observed in serum ammonia levels as well as improved cognition (Shen et al., 2015). The efficacy of FMT in Alzheimer's disease has been shown in two different Alzheimer's mouse models which showed improved spatial and recognition memory after the fecal transfer from healthy, younger donors (Sun et al., 2019); Elangovan et al., 2019; preprint). Transferring the microbiome of young wildtype (by FMT) to aged wildtype mice had also a beneficial impact on inflamm-aging, and age-associated cognitive decline (M. BOEHME et al., Program No. 676.01. 2019 Neuroscience Meeting Planner. Chicago, IL: Society for Neuroscience, 2019 online). However, in both cases the underlying mechanism was not described or further addressed.

For the diagnosis of dementia, the existing options are limited. There are many specific types and causes of dementia, which have similar but slightly different symptoms. Diagnosing dementia by symptoms alone is challenging and only possible once these symptoms have developed, i.e. often only at a very late stage of the disease. Neuro imaging techniques are often used for diagnosis but with the current technologies an early diagnosis of the disease is not possible. Some types of dementia can be diagnosed with brain biopsies, but this is very rarely recommended. Currently, cognitive testing is the gold standard for diagnosing dementia. For example, the mini mental state examination (MMSE) test is the best studied and most commonly used cognitive test for diagnosing dementia. However, cognitive tests also only allow for a diagnosis once the symptoms have developed.

For the pharmacological therapy of dementia, the existing intervention possibilities are limited. According to the guidelines of the German Society of Neurology (https://www.dgn.org/leitlinien), the use of two substances is recommended. First, acetylcholinesterase inhibitors: they are effective in terms of the ability to perform everyday activities, the improvement of cognitive functions and the overall medical impression in mild to moderate Alzheimer's dementia and treatment is recommended. Although these are constantly altered and modified, the newer acetylcholinesterase inhibitors rarely show an improved effect compared to conventional compounds (Mehta et al., 2012). Secondly, memantine: it is effective on cognition, daily function and overall clinical impression in patients with moderate to severe Alzheimer's dementia. The efficacy of memantine is not proven in mild Alzheimer's dementia. Memantine should not be used to treat patients with mild Alzheimer's dementia.

The aim of the present invention is to provide new approaches for the diagnosis and treatment of dementia diseases. In particular, the present invention aims to provide new markers for diagnosing dementia diseases, new targets for the treatment of dementia diseases and new therapeutic agents which are suitable for the treatment of dementia.

### Summary

The present invention is directed to methods for diagnosing the probability of a subject developing or having dementia, methods for screening for a drug candidate for the treatment of dementia, methods for treating a subject developing or having dementia, methods for identifying a patient group being suitable for a treatment of dementia and methods for monitoring the progress of a therapy for dementia and optionally infer a prognosis for dementia. The present invention is also directed to therapeutic agents for the treatment of dementia.

In one aspect, the invention is directed to a method for diagnosing the probability of a subject developing or having dementia comprises: receiving a sample from a subject; measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the sample; determining the probability of the subject developing or having dementia based on the concentration measured. The sample can be selected from one of a saliva sample, a urine sample, blood sample, a serum sample, a sample of brain liquor, a sample of ventricular fluid, a sample of spinal fluid, a brain tissue sample, a microbial sample, a faecal sample or a stool sample. The subject can be human. A concentration of NNN-trimethyl-5-aminovalerate between 0.005 and 0,010 µM/g creatinine in urine can be indicative for the subject developing or having dementia. The precursor of NNN-trimethyl-5-aminovalerate can be selected from one of 5-aminovalerate or N^{ε}-trimethyllysine. A concentration of 5-aminovalerate between 0.005 and 0,010 µM/g creatinine in urine or a concentration of N^{ε}-trimethyllysine between 4 and 8 µM/g creatinine in urine can be indicative for the subject developing or having dementia. The metabolite of NNN-trimethyl-5-aminovalerate can be selected from one of glutaric acid and 5-(galactosyl hydroxy)-L-lysine. A concentration of glutaric acid between 6-24 µM/g creatinine in urine, between 10-20 µM in serum, between 10-40 mM in cerebrospinal fluid, or a concentration of 5-(galactosyl hydroxy)-L-lysine between 1.4 and 2 µM/g creatinine in urine and between 0.1 and 0.3 µM in serum can be indicative for the subject developing or having dementia. The concentration can be determined by comparison to internal standards or by external comparison to metabolite standards. The concentration can be determined using any of the following methods: liquid chromatography-mass spectrometry (LC-MS), nuclear magnetic resonance (NMR) or immunoassays. Determining the probability of the subject developing or having dementia can comprise comparing the concentration with control data, in particular control data from one or more healthy individuals of the same age, same sex, same ethnicity, and/or same geographical location. The dementia can be selected from one of the following: Alzheimer's disease, Parkinson's disease, Huntington disease, frontotemporal dementia, amyotrophic lateral sclerosis, multiple sclerosis, glaucoma, myotonic dystrophy, progressive supranuclear palsy, spinal muscular atrophy, multi-system atrophy, ataxias, vascular dementia, or other dementias. The concentration can be measured in vivo with a sensor or with imaging related methods.

In a further aspect, the method for diagnosing the probability of a subject developing or having dementia comprises receiving a first sample from a subject at a first timepoint; measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the first sample; receiving a second sample from the subject at a second timepoint; measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the second sample; determining the probability of the subject developing or having dementia based on a comparison of the concentrations measured in the samples. One or more of the samples can be selected from one of a blood sample, a serum sample, a sample of brain liquor, a sample of ventricular fluid, a sample of spinal fluid, a brain tissue sample, a microbial sample, a faecal sample, a saliva sample, a urine sample or a stool sample. The subject can be human. The first and second time points can be separated by about 3-6 months. The second timepoint can be 12 to 24 weeks after the first timepoint. The second timepoint can be 3 to 6 months after the first timepoint. The method can further comprise receiving a third sample from a subject at a third timepoint; measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the third sample; determining the probability of the subject developing or having dementia based on a comparison of the concentrations measured in the samples. The second and third time point can be separated by 6-12 months. The method can comprise further samples are received at further timepoints and wherein the probability of the subject developing or having dementia is based on a comparison of the concentrations measured in the samples. The dementia can be an age-related or age-unrelated dementia. The dementia can be selected from one of the following: Alzheimer's disease, Parkinson's disease, Huntington disease, frontotemporal dementia, amyotrophic lateral sclerosis, multiple sclerosis, glaucoma, myotonic dystrophy, progressive supranuclear palsy, spinal muscular atrophy, multi-system atrophy, ataxias, vascular dementia, or other dementias. The concentration can be measured in vivo with a sensor or with imaging related methods.

In a further aspect, the method for diagnosing the probability of a subject developing or having dementia comprises receiving a sample from a subject; determining the abundance of any of Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the sample; determining the probability of the subject developing or having dementia based on the abundance measured. The sample can be selected from one or more of a microbial sample, a gut flora sample, an intestinal sample, a faecal sample and/or a stool sample. The subject can be human. The Corynebacterium can be selected from one or more of Corynebacterium glutamicum, Corynebacterium jeikeium, Corynebacterium urealyticum and Corynebacterium efficiens. The Oscillibacter can be selected from Oscillibacter valerigens and Oscillibacter sp., strain KLE 1745. The composition of the microbiota in the sample can be determined. A gut metagenome can be determined. The method can comprise comparing the abundance of the bacteria or the composition of the microbiota or the gut metagenome of the sample of the subject with a control. The control can be based on data from one or more healthy individuals. The control can be determined from one or more healthy individuals of the same age, same sex, same ethnicity, and/or same geographical location. The sample can be analyzed using any of the following methods: sequence-based techniques, genotyping assays, qPCR, RT-qPCR, clone library of full-length 16S rRNA gene sequences, DGGE, T-RFLP, ARISA, microarrays, DNA hybridization methods. The abundance of bacteria can be measured using a cell culture assay including at least one of culture in suspension or on a plate, staining, microscopy, flow cytometrical methods such as FACS, optical density measurements. The dementia can be an age-related or age-unrelated dementia. The dementia can be selected from one of the following: Alzheimer's disease, Parkinson's disease, Huntington disease, frontotemporal dementia, amyotrophic lateral sclerosis, multiple sclerosis, glaucoma, myotonic dystrophy, progressive supranuclear palsy, spinal muscular atrophy, multi-system atrophy, ataxias, vascular dementia, or other dementias.

In a further aspect, the method for diagnosing the probability of a subject developing or having dementia comprises identifying parvalbumin-positive interneurons in a sample; measuring the frequency of spontaneous IPSCs in the sample; determining the probability of the subject developing or having dementia based on the frequency measured. The sample can be selected from a brain, an acute brain slice, cultured brain tissue, a culture of neurons, or a culture of parvalbumin-positive interneurons. The subject can be human. The spontaneous IPSCs can be measured using electrophysiological methods and/or calcium imaging. The method can also comprise detecting oscillations of PV-positive GABA neurons, for example by electroencephalography (EEG) and magnetoencephalography (MEG).

In another aspect, the invention is directed to a method for screening for a drug candidate, the method comprises providing a sample including one or more of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine; subjecting the sample to a test agent; measuring the effect of the test agent on the sample; determining based on the effect of the test agent on the sample the suitability of the test agent as a drug candidate.

In another aspect, the invention is directed to a method for treating a subject developing or having dementia, the method comprising administering to the subject an agent or a combination of agents, which reduce the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the subject. The agent(s) can reduce the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in a saliva sample, a urine sample, a blood sample, a serum sample, a sample of brain liquor, a sample of ventricular fluid, a sample of spinal fluid, a brain tissue sample, a microbial sample, a faecal sample or a stool sample. The agent(s) can be inhibitors of the enzyme 5-aminopentanamidasesuch as Ba2+, Ca2+, Fe2+, Mg2+, Sn2+, Zn2+, which can be administered separately or in combination. In addition or alternatively, the agent(s) can be inhibitors of L-lysine carboxy-lyase such as 1,5-pentanediamine, 2-ethylhexyl diphenyl phosphate, 6-aminohexanoate, acridine orange, DL-alpha-difluoromethylornithine, hydroxylamine, iodoacetamide, semicarbazide, tri(2-chloro-1-(chloromethyl)ethyl) phosphate, tri(2-chloroethyl) phosphate, tri-m-cresyl phosphate, triphenyl phosphate, tris(2-chloroisopropyl)phosphate and urea, either given individually or in combination. In addition or alternatively, the agent(s) can be inhibitors of lysine 2-monooxygenase such as 2,2'-dipyridyl, 2-oxoglutarate, citrate, glutarate, oxaloacetate and succinate either applied separately or in combination.

In another aspect, the invention is directed to a method for treating a subject developing or having dementia, the method comprising administering to the subject an agent, which reduces or eradicates any of the bacteria of the phylum Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the gut flora. The agent can comprise an antimicrobial agent, a vaccine or topical probiotic intervention, or another bacterium which directly or indirectly has an influence on the abundance of the above mentioned bacteria

In another aspect, the invention is directed to a method for identifying a patient group being suitable for a treatment of dementia, the method comprising receiving a sample from a subject; measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the sample; determining the probability of the subject being responsive to a treatment based on the concentration measured. The treatment can comprise administering to the subject an agent, which reduces the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the subject.

In another aspect, the invention is directed to a method for identifying a patient group being suitable for a treatment of dementia, the method comprising receiving a sample from a subject; determining the abundance of any of Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the sample; determining the probability of the subject being responsive to a treatment based on the abundance measured. The treatment can comprise administering to the subject an agent, which reduces or eradicates any of the bacteria of the phylum Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the gut flora.

In another aspect, the invention is directed to a method for monitoring the progress of a therapy for dementia and optionally infer a prognosis comprising receiving a sample from a subject, measuring the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the sample and determining the progress of the therapy based on the concentration measured.

In another aspect, the invention is directed to a method for monitoring the progress of a therapy for dementia comprising receiving a first sample from a subject at a first timepoint, measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the first sample, receiving a second sample from the subject at a second timepoint, measuring the concentration of NNN-trimethyl-5-aminovalerate and/or precursors of NNN-trimethyl-5-aminovalerate including but not limited to 5-aminovalerate and N^{ε}-trimethyllysine (N(6),N(6),N(6)-trimethyl-L-lysine) and/or metabolites of NNN-trimethyl-5-aminovalerate in the second sample, determining the progress of the therapy based on a comparison of the concentrations measured before.

### Brief Description of the Drawings

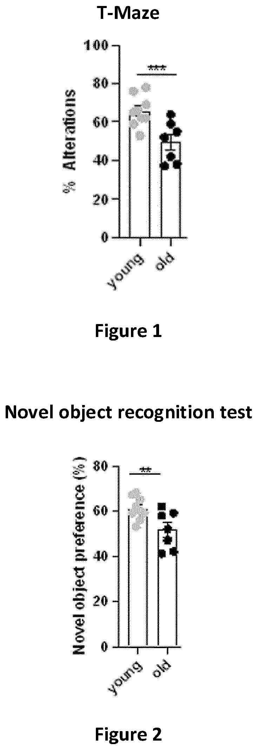

**FIG. 1** shows reduced spatial working memory (T maze test) in old mice (20-24-month-old) when compared to young mice (2-month-old). The dots represent the number of single animals. Data represented as Mean±SEM; ***P<0.001, t-test.
**FIG. 2** shows reduced recognition memory (Novel object recognition / NOR test) in old mice (20-24-month-old) when compared to young mice (2-month-old). The dots represent the number of single animals. Data represented as Mean±SEM; **P<0.01, t-test.

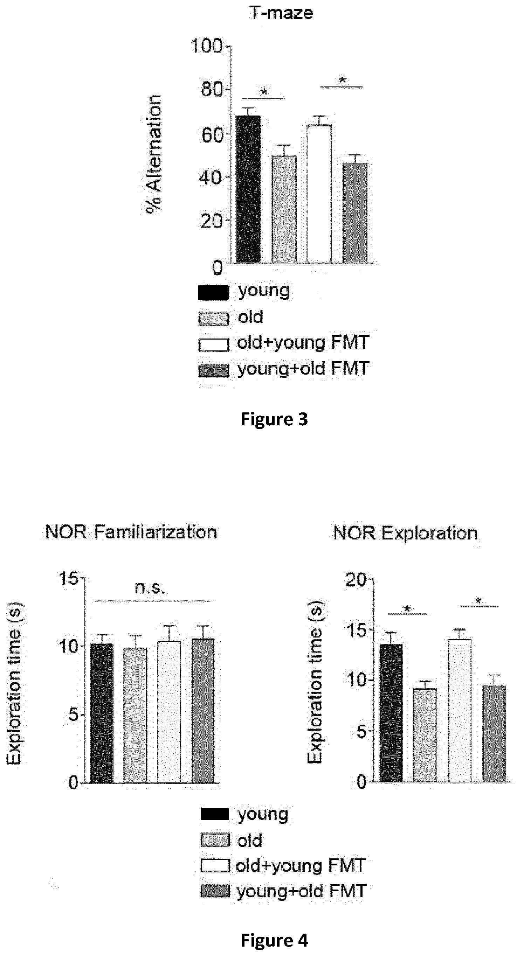

**FIG. 3** shows reduced spatial working memory (T maze test) in old mice (20-24-month-old) is improved to the level of young mice (2-month-old) after faecal microbiota transfer (FMT) from young to old mice. Spatial working memory is reduced in young mice after FMT from old mice. Data represented as Mean±SEM; *P<0.05 (n for each group = 5 mice), t-test.
**FIG. 4** shows reduced recognition memory (NOR test) in old mice (20-24-month-old) is improved to the level of young mice (2-month-old) after faecal microbiota transfer (FMT) from young to old mice. Recognition memory is reduced in young mice after FMT from old mice. Data represented as Mean±SEM; *P<0.05 (n for each group = 7 mice), t-test, ns = not significant.

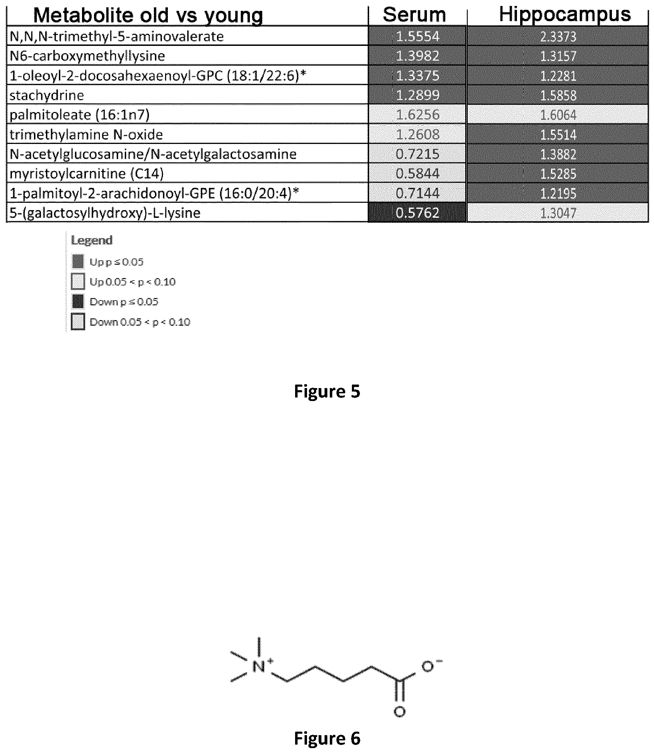

**FIG. 5** is a table which shows untargeted metabolomics of serum and hippocampal tissue from young (2 months) and old (20-24 months) mice. Numbers indicate fold-change. Greyscales represent statistical significance.
**FIG. 6** shows the structure of NNN-trimethyl-5-aminovalerate (trivial name), which is elevated in blood serum and hippocampal brain tissue of old animals when compared to young mice as analysed by mass spectrometry. IUPAC name: 5-(Trimethylammonio)- pentanoate; or δ-Valerobetaine. The molecular formula is C8H17NO2, and the average mass is 159.226 Da.

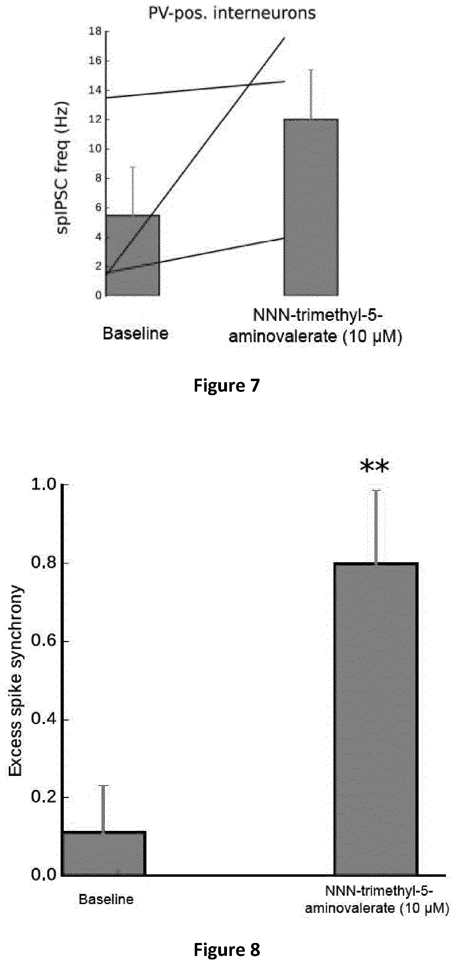

**FIG. 7** illustrates the effect of NNN-trimethyl-5-aminovalerate on parvalbumin-positive interneurons in acute mouse hippocampal slices (data from the first set of recordings are depicted, 3 sections from 3 mice were recorded, lines between bares represent data from individual mice; the experiment was performed twice in two independent runs with total recordings of 8 sections from 5 mice, both experiments showed similar results; data are represented as mean +/- SEM). The bath-applied NNN-trimethyl-5-aminovalerate induces an increase in frequencies of spontaneous IPSCs (spIPSCs) in parvalbumine-positive interneurons. This suggests a higher release probability of GABA at interneuron synapses.
**FIG. 8** shows enhanced spike-spike-synchronization after systemic application (intraperitoneally) of NNN-trimethyl-5-aminovalerate in in vivo recordings from prelimbic or infralimbic cortex of the mPFC (n = 9 mice). Data represented as Mean±SEM; **P<0.01; t-test.

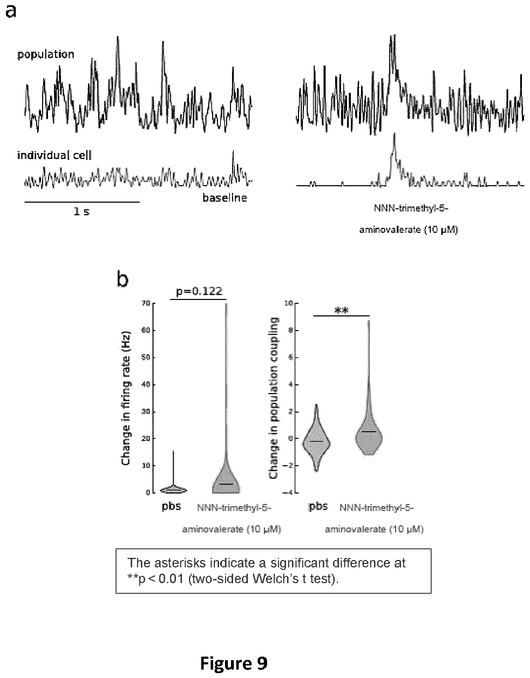

**FIG. 9a** shows single unit recordings performed from the medial prefrontal cortex during natural sleep (n=4 mice). After acquisition of baseline data, the mice were injected with NNN-trimethyl-5-aminovalerate (5 mg/kg body weight, n=4 mice, n=56 units) or with PBS only (n=4 mice, 79 units). Recording was continued 45 min after the injection.
**FIG. 9b** shows the quantification of spike rates and population coupling of all neurons. No effect of NNN-trimethyl-5-aminovalerate on average spike frequency but a significant increase in population coupling of the recorded cells (p=0.0017, Welch's test) was found. The data are normalized to the baseline values; black bars indicate means of the distribution.

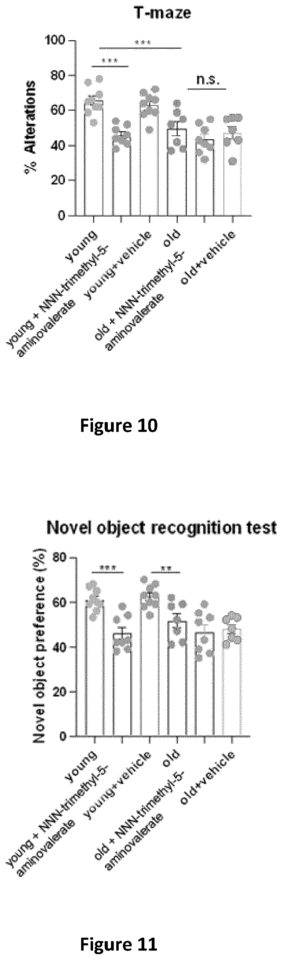

**FIG. 10** shows the effect of NNN-trimethyl-5-aminovalerate on spatial working memory (T maze test) in young mice (2-month-old) and in old mice (20-24-month-old). Injecting mice systemically with NNN-trimethyl-5-aminovalerate reduced the spatial working memory (T maze test) in young mice (2-month-old) to the level found in old mice (20-24-month-old). The same treatment had no additional impairing effects in old mice, which displayed reduced learning and memory when compared to young mice. The dots represent the number of single animals. Data represented as Mean±SEM; ***P<0.001; **P<0.01; t-test, n.s. = not significant.
**FIG. 11** shows the effect of NNN-trimethyl-5-aminovalerate on recognition memory (Novel Object Recognition test) in young mice (2-month-old) and in old mice (20-24-month-old). Injecting mice systemically with NNN-trimethyl-5-aminovalerate reduced the recognition memory (Novel Object Recognition test) in young mice (2-month-old) to the level found in old mice (20-24-month-old). The same treatment had no additional impairing effects in old mice, which displayed reduced learning and memory when compared to young mice. The dots represent the number of single animals. Data represented as Mean±SEM; ***P<0.001; **P<0.01; t-test, n.s. = not significant.

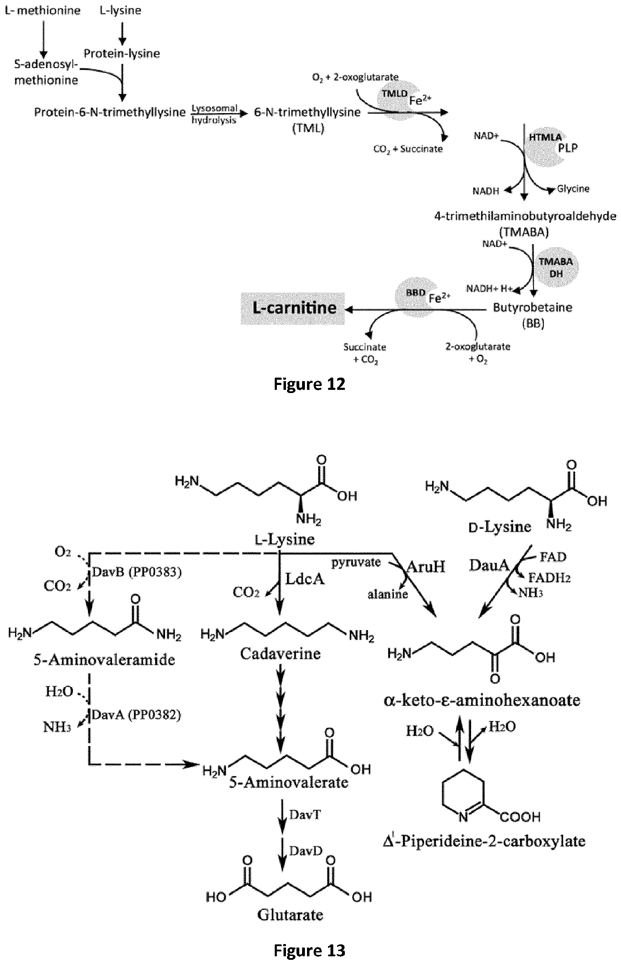

**FIG. 12** shows enzymes involved in L-lysine degradation.
**FIG. 13** shows the degradation of L-lysine.

### Detailed Description

Before the present methods and compositions are described, it is to be understood that this invention is not limited to a particular method or composition described, as such may, of course, vary. It is also to be understood that the terminology used herein is for the purpose of describing particular embodiments only, and is not intended to be limiting, since the scope of the present invention will be limited only by the appended embodiments.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and preferred methods and materials are now described. All publications mentioned herein are incorporated herein by reference to disclose and describe the methods and/or materials in connection with which the publications are cited. It is understood that the present disclosure supersedes any disclosure of an incorporated publication to the extent there is a contradiction.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

It must be noted that as used herein and in the appended embodiments, the singular forms "a", "an", and "the" include plural referents unless the context clearly dictates otherwise. Thus, for example, reference to "a cell" includes a plurality of such cells.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

Dementia is associated with the loss of cognitive functioning and behavioral abilities and is often age-dependent. To study dementia, several animal models have been established, such as the mouse model. Although animals differ with respect to humans, there is a wide consent in the field that data obtained from animal models, especially rodent models, can be transferred to humans.

A wide variety of cognitive and behavioral tests have been developed to study memory. For example, the T-maze test is a behavioral test for measuring exploratory behavior in animals, especially rodents. The T-maze test is based on the willingness of the animal to explore a new environment, i.e. they prefer to visit a new arm of the maze rather than a familiar arm. Many parts of the brain - including the hippocampus - are involved in this task. Another exemplary test is the Novel Object Recognition (NOR) task, which is used to evaluate cognition, particularly recognition memory. This test is based on the spontaneous tendency of animals to spend more time exploring a novel object than a familiar one. The choice to explore the novel object reflects the use of learning and recognition memory.

**Figures 1 and 2** show the performance of young mice (2 months) compared to old mice (20-24 months) in a T-maze test and a novel object recognition test, respectively. As can be seen, with increasing age, the memory of the mice is significantly impaired. Also, in humans, memory function often correlates with age (Nyberg et al., 2012).

In the last years, it has been shown that biochemical signaling takes place between the gastrointestinal tract and the central nervous system. In a recent Alzheimer mouse model study, it was shown that fecal microbiota transplantation (FMT) from young mice to old mice had a beneficial impact on age-associated cognitive decline (M. BOEHME et al., Program No. 676.01. 2019 Neuroscience Meeting Planner. Chicago, IL: Society for Neuroscience, 2019 online). Also, in humans, it was shown that fecal microbiota transplantation (FMT) can improve cognition (Shen et al., 2015). FMT refers to transplantation of healthy human feces to a suspected gut dysbiosis patient to regulate the intestinal microbiota. The underlying structure, which mediates the effects on brain function after FMT is the gut-brain axis (GBA), a highly complex interactive network between the gut and the brain, composed of endocrinological, immunological and neural mediators (Rhee et al., 2009). The GBA is largely mediated by the central nervous system (CNS), the enteric nervous system (ENS), and the intestinal microbiota (Grenham et al., 2011). The extrinsic nerves of the gastrointestinal (GI) tract connect the gut to the brain through vagal and spinal afferent fibers, while the brain sends efferent sympathetic and parasympathetic fibers to the GI tract (Grenham et al., 2011).

**Figures 3 and 4** show the effect of a gut transfer from young (2 months old) mice to old mice (20-24 months old) and vice versa on memory function with the T-maze test and the novel object recognition test, respectively. The transfer of gut microbiota from young (2 months old) mice to old mice (20-24 months old) improved memory of old mice (20-24 months old) in the T-maze test and the novel object recognition test. Conversely, gut transfer from old mice to young mice worsened memory function.

The object of the present invention is to identify the substances being responsible for this effect and to provide methods for diagnosing the probability of a subject developing or having dementia, methods for screening for a drug candidate for the treatment of dementia, methods for treating a subject developing or having dementia and methods for identifying a patient group being suitable for a treatment of dementia. All methods described herein are also directed for monitoring the progress of a therapy for dementia and optionally infer a prognosis, regardless of the type of therapy.

Intense metabolomic analyses have been performed to identify substances, which could be responsible for the above described effect. Therefore, blood serum and brain tissue from young mice (2 months) old mice (24 months) was analyzed using untargeted mass spectrometry (see **Figure 5**). Out of a plethora of substances, NNN-trimethyl-5-aminovalerate was identified as being significantly elevated in serum and brain of old mice when compared to young mice. The fact that it is similarly elevated in serum and brain tissue indicates that NNN-trimethyl-5-aminovalerate from the periphery can reach the brain and cross the blood-brain barrier (see **Figure 5**).

**Figure 6** shows the structure of NNN-trimethyl-5-aminovalerate (trivial name), which is elevated in blood serum and brain tissue of old animals when compared to young mice as analyzed by mass spectrometry. The IUPAC name of NNN-trimethyl-5-aminovalerate is 5-(trimethylammonio) pentanoate or δ-valerobetaine. The molecular formula of NNN-trimethyl-5-aminovalerate is C8H17NO2 and the average mass is 159.226 Da. The ChemSpider ID of NNN-trimethyl-5-aminovalerate is ID34236878.

In order to demonstrate that NNN-trimethyl-5-aminovalerate plays a pivotal role in memory impairment, several experiments have been performed. Before describing the experiments in detail, the hippocampus as well as synaptic transmission and spike-spike synchronization is briefly discussed.

The hippocampus is a brain region, which plays important roles in the consolidation of information from short-term memory to long-term memory, and in spatial memory that enables navigation. The hippocampus comprises as most other brain regions a variety of different neurons. In the following only the pyramidal cells and excitatory synaptic transmission as well as interneurons and inhibitory synaptic transmission will be briefly discussed.

Pyramidal cells are excitable cells which can be found not only in hippocampus but also e.g. in the cerebral cortex and the amygdala. The axon of a pyramidal cell is long and often extensively branched, which enables it to project over long distances. Pyramidal cell dendrites arise from the apex (apical dendrite) as well as the base of the soma (basal dendrites). Small protrusions called dendritic spines are located on the dendrites and represent the location of excitatory inputs of the neuron. Spines are extensively found in distal regions of the dendrites and are absent in proximal regions as well as the soma of pyramidal cells. The cell body and the axon-initial segment of a pyramidal cell receive only GABAergic synapses, which do not form postsynaptic spines.

Activation of excitatory postsynaptic receptors depolarizes the postsynaptic membrane due to the influx of positively charged ions into the postsynaptic cell. The postsynaptic cell starts firing, if temporal and spatial summation of excitation is powerful enough to reach the action potential threshold. Excitatory synaptic transmission thus increases the probability that the postsynaptic cell will fire an action potential. Glutamate is the dominant excitatory neurotransmitter in the central nervous system and acts via specialized metabotropic and ionotropic glutamate receptors.

Interneurons are, with few exceptions, locally projecting inhibitory neurons that regulate pyramidal cell activity. Unlike the uniform pyramidal cells, they form a diverse class of neurons that differs dramatically in innervation and firing patterns, as well as in molecular expression profiles. In the hippocampus, the prevailing inhibitory neurotransmitter released by interneurons is GABA. Different classes of interneurons have varying firing profiles and presumably release GABA at different time points to distinct subcellular domains of pyramidal cells (Klausberger and Somogyi, 2008). Often, interneurons are classified based on the presence of singular neurochemicals, such as calcium-binding proteins (parvalbumin, calretinin, and calbindin), neuropeptide Y, nitric oxide synthase, and vasoactive intestinal peptide. Interneurons are associated with several neurological disorders, such as schizophrenia, bipolar disorders, epilepsy, autism spectrum disorders, and Huntington's disease and also with memory loss.

Inhibitory synaptic transmission in adult hippocampus is mainly mediated by γ-aminobutyric acid (GABA), acting on ligand gated ionotropic GABA_{A} receptors as well as on G protein coupled GABA_{B} receptors. Inhibitory synaptic transmission controls in a spatiotemporal manner the net flow of excitability by various mechanisms, e.g. phasic and tonic modulation of the membrane potential, and shunting inhibition. Inhibition at synapses is called phasic inhibition, whereas inhibition occurring at extrasynaptic sites is called tonic. Action potential-driven GABA release acts predominantly on synaptic GABA receptors and prevents overexcitation of neurons. GABA_{A} receptors are, like most ionotropic inhibitory receptors, permeable to only one natural ion, which is chloride. Opening of the chloride channel allows chloride to cross the membrane and bring the resting membrane potential to the equilibrium potential of chloride (*E_{Cl}*), which in mature neurons is about -65 mV. Inhibition largely depends on the membrane potential as well as on the *E_{Cl}.* A more negative *E_{Cl}* compared to the resting membrane potential leads upon activation of GABA_{A} receptors to a hyperpolarizing inhibitory postsynaptic potential (IPSP). In mature neurons *E_{Cl}* is mostly close to the resting membrane potential. Activation of an inhibitory synapse in this case acts as an electrical shunt and prevents flow of positive charge to travel further. This type of inhibition is called shunting inhibition.

In the mature mammalian nervous system, synaptic transmission is mostly chemical and unidirectional. At chemical synapses, the arriving electrical signal is converted into a chemical signal, which is then able to regenerate the electrical signal in the postsynaptic neuron. Electrical signals arrive at specialized presynaptic structures, the presynaptic terminals or synaptic boutons, and trigger the release of synaptic vesicles that contain the neurotransmitter. The neurotransmitter is released into the synaptic cleft and diffuses to the juxtaposed postsynaptic membrane. Receptors in the postsynaptic membrane are activated by binding of their cognate neurotransmitter and allow ions to pass the membrane. Thereby, an electrical postsynaptic signal is generated.

The synaptic vesicles, which are located in the presynaptic terminal, fuse in a coordinated manner, which is initiated by the arriving electrical signal. However, synaptic vesicles can also fuse in a stochastic manner without any electrical activity involved. The postsynaptic signal generated by a randomly occurring fusion event (i.e. non-activity driven events) is called a miniature excitatory or inhibitory postsynaptic current (mEPSC or mIPSC). Postsynaptic signals in the presence of activity (i.e. including action potential driven events) are called spontaneous excitatory or inhibitory postsynaptic currents (spEPSC or spIPCS). Miniature and spontaneous EPSCs and IPSCs exhibit variations in amplitude, for which different mechanisms have been proposed. For example, a variation in amplitudes may be indicative for a variation in transmitter content in the synaptic vesicles, or number of postsynaptic receptors. Alterations in frequencies reflect either alterations in the release probability, or in the number of release sites.

Besides synaptic transmission another readout for brain region activities is spike-spike synchronization. Measures of spike train synchrony (or inversely spike train distances) are estimators of the (dis)similarity between two or sometimes more spike trains. Here spike train refers to a sequence of neuronal action potentials. Under the assumption that neither the shape of the action potential nor the background activity carries relevant information, neuronal responses are reduced to a spike train where the only information maintained is the timing of the individual spikes. A complementary class of approaches comprises measures of neuronal signal synchrony. Measures that estimate the degree of synchrony between spike trains are important tools for many applications. Among others, they can be used to quantify the reliability of neuronal responses upon repeated presentations of a stimulus (Mainen and Sejnowski, 1995) or to test the performance of neuronal models (Jolivet et al., 2008). Synchronous spikes are effective in triggering a spike emission in receiving neurons and have been shown to occur in relation to behavior in a number of studies on simultaneous recordings (Vaadia et al., 1995; Riehle et al., 1997; Hatsopoulos et al., 1998; Jackson et al., 2003).

**Figure 7** illustrates the effect of NNN-trimethyl-5-aminovalerate on parvalbumin-positive interneurons in acute mouse hippocampal slices (data from the first set of recordings are depicted, 3 sections from 3 mice were recorded, lines between bares represent data from individual mice; the experiment was performed twice in two independent runs with total recordings of 8 sections from 5 mice, both experiments showed similar results, , data are represented as mean +/- SEM). The bath-applied NNN-trimethyl-5-aminovalerate induces an increase in frequencies of spontaneous IPSCs (IPSCs) in parvalbumin-positive interneurons. The increased spIPSC frequency can be destructive for learning and memory (Buzsaki and Draguhn, 2004; Fries, 2015).

The fact that NNN-trimethyl-5-aminovalerate has an impact on synaptic transmission is further evidenced by *in vivo* experiments. **Figure 8** illustrates that after systemic application (intraperitoneally) of NNN-trimethyl-5-aminovalerate the spike-spike-synchronization is enhanced. This was observed by *in vivo* recordings from prelimbic or infralimbic cortex of the medial prefrontal cortex (n = 9 mice, data represented as mean ± SEM; **P<0.01; t-test). Spike synchronization quantifies the degree of synchrony from the relative number of quasi-simultaneous appearances of spikes. It is zero if and only if the spike trains do not contain any coincidences and reaches one if and only if each spike in every spike train has one matching spike in all the other spike trains. The enhanced spike-spike synchronization alters the finely tuned balance of excitation and inhibition and impairs cognitive functions. The basis for this assumption is that efficient neuronal coordination between brain regions across the entire brain is necessary for cognition. A proposed mechanism for such coordination is oscillatory synchronization; that is, populations of neurons transmit information by coordinating their oscillatory activity with the oscillations of the receptor population at certain frequencies. Furthermore, different frequencies, or, more generally, different oscillatory patterns, subserve different functions. At the same time, phase-coupling between neuronal populations in specific frequency bands has been proposed as a mechanism for regulating the integration and flow of cognitive content (Buzsaki and Draguhn, 2004; Fries, 2015).

The pivotal role of NNN-trimethyl-5-aminovalerate in synaptic transmission is further evidenced by in vivo single unit recordings from the medial prefrontal cortex. The prefrontal cortex is the cerebral cortex, which covers the front part of the frontal lobe. This brain region has been implicated in planning complex cognitive behavior, personality expression, decision making and moderating social behavior. There is growing evidence that the PFC is involved not only in frontal lobe-type dementias, but also Alzheimer disease, mild cognitive impairment, and normal aging. It may be considered that different parts of the PFC are related to different memory types and memory dysfunctions (Maclin et al., 2019; Mizoguchi et al., 2009).

**Figure 9** shows an enhanced population coupling upon systemic application of NNN-trimethyl-5-aminovalerate. Single unit recordings were performed from the medial prefrontal cortex during natural sleep (n=4 mice). After acquisition of baseline data, the mice were injected with NNN-trimethyl-5-aminovalerate (5 mg/kg body weight, n = 4 mice, n = 56 units) or with phosphate-buffered saline (PBS) as a control (n = 4 mice, n = 79 units). Recording was continued 45 minutes after the injection. **Figure 9a** shows in the upper part an example of a short segment of the population firing rate of all units of one recording session (binned in 100 ms time epochs) and in the bottom part the binned spike rate of one neuron before (left side) and after (right side) the injection of NNN-trimethyl-5-aminovalerate. An enhanced synchronization in the prefrontal network can be observed. **Figure 9b** shows the quantification of spike rates and population coupling of all neurons and revealed that NNN-trimethyl-5-aminovalerate does not have an effect on average spike firing rate but significantly increased population coupling of the recorded cells (p = 0.0017, Welch's t-test, data shown are normalized to the baseline values, black bars indicate means of the distribution). An increase of population coupling disrupts the normal function of the neuronal network and impairs cognitive function. Thus, NNN-trimethyl-5-aminovalerate can be responsible for impaired memory and causative for dementia.

The effect of NNN-trimethyl-5-aminovalerate on memory function is further evidenced in behavioral tests.

**Figure 10** shows that injecting mice systemically (intraperitoneally) with NNN-trimethyl-5-aminovalerate reduced the spatial working memory (T maze test) in young mice (2-month-old) to the level found in old mice (20-24-month-old). The same treatment had no additional impairing effects in old mice, which displayed reduced learning and memory when compared to young mice (dots represent the number of single animals, data represented as mean ± SEM; ***P<0.001; **P<0.01; t-test, n.s. = not significant). PBS was injected as a control to assess the possibility that dilution of negative factors present in the circulation could underlie cognitive improvement.

Similarly, **Figure 11** shows that injecting mice systemically (intraperitoneally) with NNN-trimethyl-5-aminovalerate also reduced the recognition memory (Novel Object Recognition test) in young mice (2-month-old) to the level found in old mice (20-24-month-old). The same treatment had no additional impairing effects in old mice, which displayed reduced learning and memory when compared to young mice (dots represent the number of single animals, data represented as mean ± SEM; ***P<0.001; **P<0.01; t-test, n.s. = not significant). PBS was again injected as a control to assess the possibility that dilution of negative factors present in the circulation could underlie cognitive improvement.

Thus, behavioral tests also show that NNN-trimethyl-5-aminovalerate has the potential to disrupt normal brain functioning in young mice to the level of old mice.

Thus, increased levels of NNN-trimethyl-5-aminovalerate are found in serum and brain of old animals when compared to young animals. NNN-trimethyl-5-aminovalerate modulates inhibitory synaptic transmission and network activity and administering of NNN-trimethyl-5-aminovalerate to young and healthy animals results in cognitive impairment. NNN-trimethyl-5-aminovalerate thus correlates with normal brain function and elevated levels of NNN-trimethyl-5-aminovalerate are indicative for an impaired memory and/or the presence or development of dementia.

NNN-trimethyl-5-aminovalerate, its precursors and metabolites are markers for diagnosing the probability of a subject developing or having dementia.

The method for diagnosing the probability of a subject developing or having dementia can comprise receiving a sample from a subject, which can be a human. Receiving the sample does not include any step practiced on the human or animal body. Rather, receiving is meant as being provided with a sample from the subject. The sample can be one or more of a saliva sample, a urine sample, a blood sample, a serum sample, a sample of brain liquor, a sample of ventricular fluid, a sample of spinal fluid, a brain tissue sample, a microbial sample, a faecal sample or a stool sample or any other suitable sample.

The method can comprise measuring the absolute concentration of NNN-trimethyl-5-aminovalerate and/or precursors of NNN-trimethyl-5-aminovalerate and/or metabolites of NNN-trimethyl-5-aminovalerate in the sample. There are two standard ways to determine absolute concentrations: by comparison to internal standards (for liquid chromatography-mass spectrometry (LC-MS), this is accomplished by measuring intensity difference between ¹³C or ¹⁵N labeled standards and unlabeled metabolites; for nuclear magnetic resonance (NMR), a single reference metabolite can frequently be used) or by external comparison to metabolite standards prepared at a range of concentrations. To account for matrix effects, external calibration curves are preferably made by adding standards into samples. To equate losses during extraction and handling between standards and endogenous analyte, standards should be added in the original extraction solvent, not to the final samples. An important constraint in LC-MS is the time required for each chromatography run. Accordingly, there is substantial interest in direct MS to increase sample throughput. One commercial example of a chromatography-free system is the RapidFire instrument from Agilent. The benefit is the analysis time of < 1 min per sample, versus approximately 30 min per sample with typical metabolomics LC-MS methods. Immunoassays, which are antibody-based analytical methods for quantitative/qualitative analysis (e.g. enzyme immunoassay/enzyme-linked immunosorbent assay (ELISA)) are another option to determine concentrations.

The method can further comprise determining the probability of the subject developing or having dementia based on the concentration measured. Therefore, the concentration of NNN-trimethyl-5-aminovalerate and/or precursors of NNN-trimethyl-5-aminovalerate and/or metabolites of NNN-trimethyl-5-aminovalerate can be determined by e.g. a targeted metabolomic approach or by ELISA. The concentration can be compared to internal standards or to metabolite standards. Metabolite standards can be established through the analysis of control datasets. Therefore, the concentration can be compared with healthy controls or with controls having dementia. The control can be based on data from one or more individuals. The control can be determined from one or more individuals of the same age, same sex, same ethnicity, and/or same geographical location. Comparing the concentration with control data of patients having dementia may be beneficial for the prognosis of dementia. A predictive model for each patient can be developed based on the decision tree that includes the main parameters: age, sex, ethnicity and geographical location of the patient, NNN-trimethyl-5-aminovalerate and precursors of NNN-trimethyl-5-aminovalerate (e.g. 5-aminovalerate) concentrations. Multivariante receiver operating characteristic (ROC) analysis can be used to develop a decision tree to determine the diagnostic power in differentiating between older adults who are healthy and cognitively normal, those with mild cognitive impairment (MCI) and those with Alzheimer's disease (AD).

In general, concentrations of any substances herein are considered increased if the increase is compared to control values at least 10%, at least 20%, at least 30%, at least 40%, at least 50%, at least 60%, at least 70%, at least 80%, at least 90%, 100%, 200%, 300% or 1000%, including all the percentages between 10-1000% . An increased concentration can be predictive for a subject developing or having dementia. The higher the increase is, the more reliable the prediction is.

In urine samples, the concentration of NNN-trimethyl-5-aminovalerate can be normalized to creatinine levels. A concentration of NNN-trimethyl-5-aminovalerate between 0.005 and 0,010 µM/g creatinine in urine can be indicative for the subject developing or having dementia. The creatinine levels can also be adjusted to sex and subject-specific parameters (e.g. to account for elevated or reduced levels of creatinine).

The concentration of precursors or metabolites of NNN-trimethyl-5-aminovalerate can also be indicative for the probability of the subject developing or having dementia. The precursor of NNN-trimethyl-5-aminovalerate can be selected from one or more of 5-aminovalerate or N^{ε}-trimethyllysine (N(6),N(6),N(6)-trimethyl-L-lysine).

NNN-trimethyl-5-aminovalerate can be produced in vivo or in vitro by methylation of 5-aminovalerate:

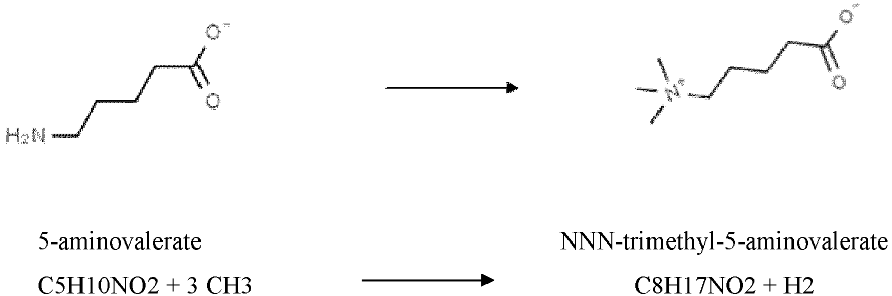

NNN-trimethyl-5-aminovalerate can also be produced in vivo or in vitro by a reaction of glycine together with Nε-trimethyllysine:

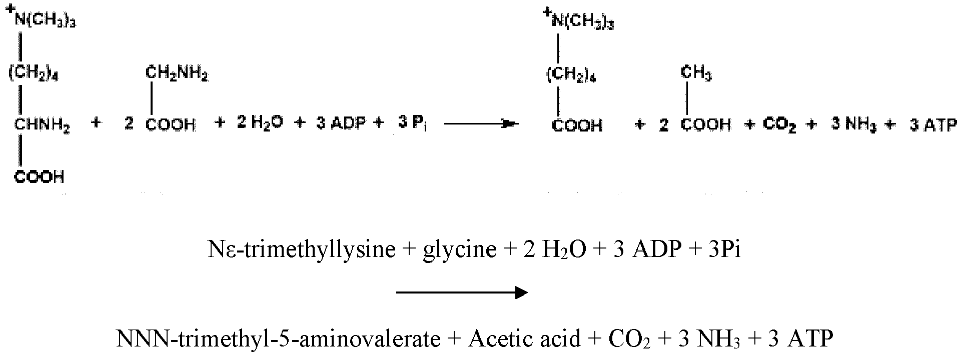

Nε-trimethyllysine (N(6),N(6),N(6)-trimethyl-L-lysine) is a metabolite, which is generated during L-lysine degradation and the ultimate synthesis of L-carnitine. As can be seen in **Figure 12****,** at least five enzymes are involved in the overall biosynthetic pathway, namely 6-N-trimethyllysine dioxygenase (TMLD), 4-trimethylaminobutyraldehyde dehydrogenase (TMABADH), serine hydroxymethyltransferase 1 and 2 (SHMT1 and 2) and γ-butyrobetaine hydroxylase (BBH).

As can be seen in **Figure 13**, 5-aminovalerate (or 5-aminopentanoic acid) is a lysine degradation product. It can be produced through bacterial catabolism of lysine. In microbes, lysine catabolism can be initiated either through monooxygenase, decarboxylase, or transaminase activities. I-lysine utilization might be mediated by the lysine decarboxylase pathway with cadaverine and 5-aminovalerate as intermediates. Alternatively, conversion of l-lysine into 5-aminovalerate may also be accomplished by a coupled reaction catalyzed by AruH and AruI. The AruH and Arul enzymes were reported as arginine:pyruvate transaminase and 2-ketoarginine decarboxylase, respectively (Yang and Lu, 2007).

5-(galactosyl hydroxy)-L-lysine is a Glycoside of 5-hydroxylysine. 5-Hydroxylysine is an amino acid with the molecular formula C 6 H 14 N 2 O 3. It is a hydroxy derivative of lysine.

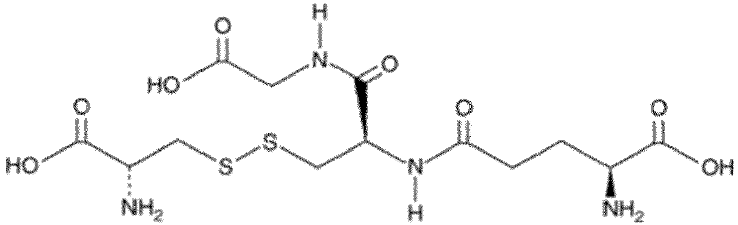

Concentrations of 5-aminovalerate in healthy individuals, which do not show symptoms of dementia can be in the range of 262 +/- 254 nmol/g wet feces and 0.1-2 µmol/mmol creatinine in urine. Concentrations of 5-aminovalerate, which are significantly higher or lower than seen in healthy controls can be indicative for the subject developing or having dementia.

Concentrations of N^{ε}-trimethyllysine in healthy individuals, which do not show symptoms of dementia can be in the range of 1.5 +/- 2.0 µM in cerebrospinal fluid and of 5.3 (3.8-10.4) µmol/mmol creatinine in urine. A concentration of N^{ε}-trimethyllysine, which is significantly higher or lower than seen in healthy controls can be indicative for the subject developing or having dementia.

The concentration of metabolites of NNN-trimethyl-5-aminovalerate can also be indicative for the probability of the subject developing or having dementia. The metabolite of NNN-trimethyl-5-aminovalerate can be selected from one of glutaric acid and 5-(galactosyl hydroxy)-L-lysine.

Concentrations of glutaric acid in healthy individuals, which show no signs of dementia can be in the range of 0.8 (0.0-1.8) µM in blood, 0-1 µM in cerebrospinal fluid, 189 +/- 125 nmol/g wet feces, 0.5 +/- 1.4 µM in saliva. Concentrations of 5-(galactosyl hydroxy)-L-lysine in healthy individuals, which show no signs of dementia can be in the range of 2.5 (1-5) umol/mmol creatinine in urine. A concentration of glutaric acid and of 5-(galactosyl hydroxy)-L-lysine, which is significantly higher or lower can be indicative for the subject developing or having dementia.

The dementia can be selected from one of the following: Alzheimer's disease, Parkinson's disease, Huntington disease, frontotemporal dementia, amyotrophic lateral sclerosis, multiple sclerosis, glaucoma, myotonic dystrophy, progressive supranuclear palsy, spinal muscular atrophy, multi-system atrophy, ataxias, vascular dementia, dementia Lewy body or other dementias.

The concentration can also be measured in vivo with a sensor or with imaging related methods. Continuous monitoring of NNN-trimethyl-5-aminovalerate and/or precursors and/or metabolites has the potential to greatly improve cognitive impairment management. A device which performs minimally invasive metabolite sensing in the blood or in the cerebrospinal fluid or in any other suitable sample can be based on the integration of an ultra-miniaturized electrochemical sensing probe, e.g. in the lumen of a single hollow microneedle, separately realized using e.g. standard silicon microfabrication methods. By placing the sensing electrodes inside the lumen facing an opening towards the blood vessel lumen or the spinal fluid, real-time measurement purely can be performed relying on molecular diffusion over a short distance. Furthermore, the device relies only on passive capillary lumen filling without the need for complex fluid extraction mechanisms. The combination of sensor technology with microneedles for reliable insertion and injection provides the possibility to correctly and dynamically track NNN-trimethyl-5-aminovalerate its precursors or metabolites over time.

In addition or alternatively, the method for diagnosing the probability of a subject developing or having dementia can comprises receiving a first sample from a subject at a first timepoint; measuring the concentration of NNN-trimethyl-5-aminovalerate and/or precursors of NNN-trimethyl-5-aminovalerate and/or metabolites of NNN-trimethyl-5-aminovalerate in the first sample; receiving a second sample from the subject at a second timepoint; measuring the concentration of NNN-trimethyl-5-aminovalerate and/or precursors of NNN-trimethyl-5-aminovalerate and/or metabolites of NNN-trimethyl-5-aminovalerate in the second sample; determining the probability of the subject developing or having dementia based on a comparison of the concentrations measured in the samples. As above, one or more of the samples can be selected from one of a blood sample, a serum sample, a sample of brain liquor, a sample of ventricular fluid, a sample of spinal fluid, a brain tissue sample, a microbial sample, a faecal sample, a saliva sample, a urine sample, stool sample or any other suitable sample. The subject can be human.

Comparing the concentrations at different timepoints has the benefit that no reference data of healthy individuals as a control is needed. Basically, the first timepoint serves as the baseline or control value and may be specific for the subject. However, the concentrations of the first sample and second sample (and any further sample) may still be compared to control values of healthy individuals, which may further improve the accuracy of the prediction.

The first and second time points can be separated by about 3-6 months. The second timepoint can be 12 to 24 weeks after the first timepoint. The second timepoint can be 3 to 6 months after the first timepoint. Following the indicated time-points will allow to detect general trends and can exclude individual variability. Some biochemical compounds, such as oleoylethanolamide, melatonin, and dopamine, are influenced by circadian rhythms, making the time of day of sample withdrawal important. Knowing the length of time that a sample is stable at freezing temperatures is important for longitudinal studies in which samples are stored for long periods and analyzed simultaneously with samples stored for short periods to minimize inter-assay variability.

The method can further comprise receiving a third sample from a subject at a third timepoint; measuring the concentration of NNN-trimethyl-5-aminovalerate and/or precursors of NNN-trimethyl-5-aminovalerate and/or metabolites of NNN-trimethyl-5-aminovalerate in the first sample; determining the probability of the subject developing or having dementia based on a comparison of the concentrations measured in the samples. The second and third time point can be separated by 6-12 months. The method can comprise further samples are received at further timepoints and wherein the probability of the subject developing or having dementia is based on a comparison of the concentrations measured in the samples.

It may be beneficial to the accuracy of the prediction to assess samples, which have been taken in the same context (e.g. same dietary status, same timepoint, etc.). This may reduce naturally occurring fluctuations of the concentration(s).

As above, the dementia can be an age-related or age-unrelated dementia. The dementia can be selected from one of the following: Alzheimer's disease, Parkinson's disease, Huntington disease, frontotemporal dementia, amyotrophic lateral sclerosis, multiple sclerosis, glaucoma, myotonic dystrophy, progressive supranuclear palsy, spinal muscular atrophy, multi-system atrophy, ataxias, vascular dementia, dementia Lewy body or other dementias. As above, the concentration can also be measured in vivo with a sensor or with imaging related methods.

In addition, or alternatively, the method for diagnosing the probability of a subject developing or having dementia can comprise an analysis of the gut flora of the subject.

Instead of determining the concentration of NNN-trimethyl-5-aminovalerate itself, it is also possible to determine the presence and/or abundance of specific gut bacteria, which are involved in its synthesis. For example, 5-aminovalerate, a precursor of NNN-trimethyl-5-aminovalerate, can be found in Corynebacterium. Furthermore, lysine degradation, which leads to the production of NNN-trimethyl-5-aminovalerate, occurs in Corynebacterium, a bacterium, which is found in the gut flora of humans. NNN-trimethyl-5-aminovalerate is produced during lysine degradation. Further lysine fermenting bacteria in the human gut are Escherichia coli, Klebsiella pneumoniae, Clostridium bifermentans, Clostridium sporogenes, Clostridium sticklandii, Clostridioides difficile and Clostridium perfringens (Dai et al., 2011).

The method for diagnosing the probability of a subject developing or having dementia can thus comprise receiving a sample from a subject; determining the abundance of any of Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensis in the sample; determining the probability of the subject developing or having dementia based on the abundance measured.

Determining the abundance is understood as detecting whether the bacteria are present and does not necessarily require to detect the quantitative amount. The quantity of the bacteria, however, may be a factor which improves the prediction of the probability of the subject developing or having dementia.

The sample can be selected from one or more of a microbial sample, a gut flora sample, an intestinal sample, a faecal sample and/or a stool sample. The subject can be human.

The Corynebacterium can be selected from one or more of Corynebacterium glutamicum, Corynebacterium jeikeium, Corynebacterium urealyticum and Corynebacterium efficiens.

The Oscillibacter can be selected from Oscillibacter valerigens and Oscillibacter sp., strain KLE 1745.

The composition of the microbiota in the sample can be determined. A gut metagenome can be determined. The method can comprise comparing the abundance of the bacteria or the composition of the microbiota or the gut metagenome of the sample of the subject with a control. The control can be based on data from one or more healthy individuals. The control can be determined from one or more healthy individuals of the same age, same sex, same ethnicity, and/or same geographical location.

The gut metagenome or microbiota compositional alterations of the gut metagenome or microbiota can be identified between disease or risk subjects (patients) and control subjects, preferably this is carried out by identifying alterations in the type or number of bacterial groups or species which are present, for example an alteration in species or other taxonomical abundance. Preferred methods comprise analyzing a sample of gut flora from said subject for the presence of specific bacterial groups or species, for example analyzing said sample for the enrichment or increase in certain bacterial groups or species compared to a control level, in particular those species which have been identified as being enriched or increased in the gut metagenome of subjects with dementia or dementia associated disease. Alternatively, for bacterial groups or species that have been identified as being enriched or increased in the gut metagenome of control subjects (healthy subjects), the sample, e.g. the gut flora sample, from the test subject might be analyzed for the reduction or depletion in certain bacterial groups or species, compared to a control level. The "increase" in the levels or "increased" level or "enrichment", etc., of bacteria or genes as described herein includes any measurable increase or elevation or enrichment of the marker in question when the marker in question is compared with a control level. Preferably the increase in level will be significant, more preferably clinically or statistically significant (preferably with a probability value of <0.05), most preferably clinically and statistically significant (preferably with a probability value of <0.01). The sample can be analyzed using any of the following methods: sequence-based techniques, genotyping assays, qPCR, RT-qPCR, clone library of full-length 16S rRNA gene sequences, DGGE, T-RFLP, ARISA, microarrays, DNA hybridization methods. The abundance of bacteria can be measured using a cell culture assay including at least one of culture in suspension or on a plate, staining, microscopy, flow cytometrical methods such as FACS, optical density measurements.

For example, a gut flora sample such as a fecal sample or intestinal sample could be analyzed at the nucleic acid level by sequence-based techniques. This invention can be practiced for example by using barcoded multiplexed-454 sequencing to analyze the bacterial composition of the gut microbiota, alone or in combination with other analysis. The invention can also be practiced using other methods for quantification of specific bacterial species or groups known in the art.

As above, the dementia can be an age-related dementia and can be selected from one of the following: Alzheimer's disease, Parkinson's disease, Huntington disease, frontotemporal dementia, amyotrophic lateral sclerosis, multiple sclerosis, glaucoma, myotonic dystrophy, progressive supranuclear palsy, spinal muscular atrophy, multi-system atrophy, ataxias, vascular dementia, dementia Lewy body or other dementias.

In addition, or alternatively, the method for diagnosing the probability of a subject developing or having dementia comprises identifying parvalbumin-positive interneurons in a sample; measuring the frequency of spontaneous IPSCs in the sample; determining the probability of the subject developing or having dementia based on the frequency measured.

As discussed above in detail, NNN-trimethyl-5-aminovalerate has an effect on the frequency of spontaneous IPSCs in parvalbumin-positive interneurons. An abnormal spIPSC frequency can therefore also be indicative for a subject developing or having dementia.

Similarly, the method for diagnosing the probability of a subject developing or having dementia can comprise of identifying parvalbumin-positive interneurons in a sample; measuring oscillations of parvalbumin-positive interneurons; determining the probability of the subject developing or having dementia based on oscillations measured.

As shown above, NNN-trimethyl-5-aminovalerate has an effect on PV-positive GABA neurons, which play a key role in the production of gamma oscillations (Buzsaki and Wang, 2012). Determining and/or quantifying oscillations may therefore predict the probability of the subject developing or having dementia. The oscillations may be detected by electroencephalography (EEG) and magnetoencephalography (MEG).

The sample can be selected from a brain, an acute brain slice, cultured brain tissue, a culture of neurons, or a culture of parvalbumin-positive interneurons. The subject can be human. The spontaneous IPSCs can be measured using electrophysiological methods and/or calcium imaging. The method can also comprise detecting oscillations of PV-positive GABA neurons, for example by electroencephalography (EEG) and magnetoencephalography (MEG).

In addition or alternatively, the invention is directed to a method for screening for a drug candidate, the method comprises providing a sample including one or more of NNN-trimethyl-5-aminovalerate, precursors of NNN-trimethyl-5-aminovalerate, metabolites of NNN-trimethyl-5-aminovalerate; subjecting the sample to a test agent; measuring the effect of the test agent on the sample; determining based on the effect of the test agent on the sample the suitability of the test agent as a drug candidate. The sample can be selected from one of a saliva sample, a urine sample, a blood sample, a serum sample, a sample of brain liquor, a sample of ventricular fluid, a sample of spinal fluid, a brain tissue sample, a microbial sample, a faecal sample or a stool sample. The test agent can have a mechanism of action which involves a specific biochemical interaction with any of NNN-trimethyl-5-aminovalerate, precursors of NNN-trimethyl-5-aminovalerate, metabolites of NNN-trimethyl-5-aminovalerate, such as enzymatic degradation, chelating, binding, absorption or the like. The effect of the test agent may be a reduction of the concentration of any of NNN-trimethyl-5-aminovalerate, precursors of NNN-trimethyl-5-aminovalerate, metabolites of NNN-trimethyl-5-aminovalerate. The suitability of the test agent as a drug candidate may be determined based on the effect of the test agent on reducing the concentration of any of NNN-trimethyl-5-aminovalerate, precursors of NNN-trimethyl-5-aminovalerate, metabolites of NNN-trimethyl-5-aminovalerate. In addition or alternatively, the test agent can also reduce or eradicate any of the bacteria of the Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the sample. The test agent can comprise an antimicrobial agent, a vaccine or topical probiotic intervention, or another bacterium which directly or indirectly has an influence on the abundance of the above mentioned bacteria. For example, other bacteria such as E. coli may be increased which results in a decrease of the above mentioned bacteria. The suitability of the test agent as a drug candidate may be determined based on the effect of the test agent on the the abundance of any of the above mentioned bacteria.
As discussed above, NNN-trimethyl-5-aminovalerate has an effect on synaptic transmission, in particular on inhibitory transmission. Alterations in frequencies of spontaneous IPSCs indicate either alterations in the release probability, or in the number of release sites. NNN-trimethyl-5-aminovalerate may also act as a GABA receptor agonist. Any agent, which reduces the effect of NNN-trimethyl-5-aminovalerate on synaptic transmission may have the potential to decrease the symptoms of dementia or to delay the onset of dementia. One potential mechanism is the normalization of spike-spike frequency synchronization. This will improve the information processing within a given brain region, like in this case the PFC. At the same time the synchronization with other brain regions (e.g. hippocampus) will be improved. As a consequence, cognitive processing will be facilitated.

The invention is also directed to various methods for treating a subject developing or having dementia. By "treatment", "treating" and the like it is generally meant obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment" as used herein covers any treatment of a disease in humans and includes: (a) preventing the disease from occurring in a subject which may be predisposed to the disease but has not yet been diagnosed as having it; (b) inhibiting the disease, i.e., arresting or decelerating its development; or (c) relieving the disease, i.e., causing regression of the disease. Treatment may result in a variety of different physical manifestations, e.g., modulation in gene expression, rejuvenation of tissue or organs, etc. The therapeutic agent may be administered before, during or after the onset of disease or injury. The treatment of ongoing disease, where the treatment stabilizes or reduces the undesirable clinical symptoms of the patient, is of particular interest. Such treatment may be performed prior to complete loss of function in the affected tissues. The therapeutic agent may be administered during the symptomatic stage of the disease, and in some cases after the symptomatic stage of the disease. The progress of the therapy may be monitored or evaluated or determined by measuring the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the subject. The methods described above or below in context with diagnosing equally apply to the method of monitoring or evaluating or determining the progress of a therapy and optionally infer a prognosis. The therapy may be a therapy, which acts on any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the subject but can also be any other therapy. Any therapeutic treatment of dementia can result in a change of the concentration of any of these substances and consequently, these concentrations may be used for monitoring or evaluating or determining the progress of the therapy.

For example, the invention is directed to a method for treating a subject developing or having dementia, the method comprising administering to the subject an agent or a combination of agents, which reduce the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the subject. The agent(s) can reduce the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in a saliva sample, a urine sample, a blood sample, a serum sample, a sample of brain liquor, a sample of ventricular fluid, a sample of spinal fluid, a brain tissue sample, a microbial sample, a faecal sample or a stool sample. As shown in the enzymatic reactions below, the agent(s) can be inhibitors of the enzyme 5-aminopentanamidase, which causes the production of 5-aminovalerate from 5-aminopentanamide. This enzyme can be inhibited by the following compounds, which might be administered separately or in combination: Ba2+, Ca2+, Fe2+, Mg2+, Sn2+, Zn2+. Another target would be L-lysine carboxy-lyase, which generates cadaverine from L-lysine. This enzyme can be inhibited by 1,5-pentanediamine, 2-ethylhexyl diphenyl phosphate, 6-aminohexanoate, acridine orange, DL-alpha-difluoromethylornithine, hydroxylamine, iodoacetamide, semicarbazide, tri(2-chloro-l-(chloromethyl)ethyl) phosphate, tri(2-chloroethyl) phosphate, tri-m-cresyl phosphate, triphenyl phosphate, tris(2-chloroisopropyl)phosphate and urea, either given individually or in combination. The production of 5-aminopentanamide from L-lysine can be targeted by inhibiting the enzyme lysine 2-monooxygenase. Suitable inhibitors of this enzymatic reaction are 2,2'-dipyridyl, 2-oxoglutarate, citrate, glutarate, oxaloacetate and succinate either applied separately or in combination.

### Enzymatic Reactions:

| |
|---|
| **Reaction:** 5-aminopentanamide + H2O = 5-aminovalerate + NH3 |
| **Enzyme:** 5-aminopentanamidase |
| **Inhibitor:** Ba2+, Ca2+, Fe2+, Mg2+, Sn2+, Zn2+ |
| **Reaction:** L-lysine <=> cadaverine + CO2 |
| **Enzyme:** L-lysine carboxy-lyase |
| **Inhibitor:** 1,5-pentanediamine, 2-ethylhexyl diphenyl phosphate, 6-aminohexanoate, acridine orange, DL-alpha-difluoromethylornithine, hydroxylamine, iodoacetamide, semicarbazide, tri(2-chloro-1-(chloromethyl)ethyl) phosphate, tri(2-chloroethyl) phosphate, tri-m-cresyl phosphate, triphenyl phosphate, tris(2-chloroisopropyl)phosphate, urea |
| **Reaction:** L-lysine + O2 = 5-aminopentanamide + CO2 + H2O |
| **Enzyme:** lysine 2-monooxygenase |
| **Inhibitor:** 2,2'-dipyridyl, 2-oxoglutarate, citrate, glutarate, oxaloacetate, succinate |

NNN-trimethyl-5-aminovalerate is produced as part of the catabolism of lysine to succinate. Lysine enters the pathway via 5-aminovalerate by the promiscuous enzymes GabT and GabD. GabT is described as GABA transaminase that yields succinic semialdehyde. GabD is a dehydrogenase that converts succinic semialdehyde to succinate (Schneider et al., 2002). Repression of the pathway is possible by introducing CsiR, which encodes for a ligand-dependent transcription factor that represses the CsiD operon, into gut microbiota, e.g. by a viral construct (Knorr et al., 2018).

The invention is also directed to a method for treating a subject developing or having dementia, the method comprising administering to the subject an agent, which reduces or eradicates any of the bacteria of the Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the gut flora. The agent can comprise an antimicrobial agent, a vaccine or topical probiotic intervention, or another bacterium which directly or indirectly has an influence on the abundance of the above mentioned bacteria. For example, other bacteria such as E. coli may be increased which results in a decrease of the above mentioned bacteria.

The agent may be delivered as a formulation, a pharmaceutical or a pharmaceutical preparation or a food supplement, which may be formulated as a delayed or gradual enteric release composition or formulation or an immediate release formulation. Optionally the formulation comprises a gastro-resistant coating designed to dissolve at a pH of 7 in the terminal ileum, e.g., an active ingredient is coated with an acrylic based resin or equivalent, e.g., a poly(meth)acrylate, e.g. a methacrylic acid copolymer B, NF, which dissolves at pH 7 or greater, e.g., comprises a multimatrix (MMX) formulation.

The formulation, the pharmaceutical or the pharmaceutical preparation further comprises an additional antimicrobial or antibiotic, wherein optionally the additional antimicrobial or antibiotic comprises: an ampicillin, a sulbactama tetracycline, a cephalosporin, a carbapenem, an imipenem, a meropenem, a monobactam, a lincosamide, a clindamycin, a quinolone, a fluoroquinolone, a sulphonamide, a fradicin, a nitroimidazole, a metronidazole, a tinidazole, an anti-clostridial agent, or a ramoplanan, an aminoglycoside antibiotic, a gentamycin, a neomycin, a streptomycin, a paromomycin, a verdamicin, a mutamicin, a sisomicin, a netilmicin, a retymicin, a kanamycin, an amphenicol, an ansamycin, a beta-lactam (β-lactam) antibiotic, a carbapenem, a cephalosporin, a cephamycin, a monobactam, an oxacephem, a lincosamide antibiotic, a clindamycin.

In addition or alternatively, the invention is directed to a method for identifying a patient group being suitable for a treatment of dementia, the method comprising receiving a sample from a subject; measuring the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the sample; determining the probability of the subject being responsive to a treatment based on the concentration measured. The treatment can comprise administering to the subject an agent, which reduces the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the subject.

In addition or alternatively, the invention is directed to a method for identifying a patient group being suitable for a treatment of dementia, the method comprising receiving a sample from a subject; determining the abundance of any of Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the sample; determining the probability of the subject being responsive to a treatment based on the abundance measured. The treatment can comprise administering to the subject an agent, which reduces or eradicates any of the bacteria of the phylum Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the gut flora. The agent can comprise an antimicrobial agent, a vaccine or topical probiotic intervention, or another bacterium which directly or indirectly has an influence on the abundance of the above mentioned bacteria. For example, other bacteria such as E. coli may be increased which results in a decrease of the above mentioned bacteria.

In addition or alternatively, the invention is also directed to a method for monitoring the progress of a therapy for dementia and optionally infer a prognosis comprising receiving a sample from a subject, measuring the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the sample and determining the progress of the therapy based on the concentration measured. The therapy may be a therapy, which acts on any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the subject but can also be any other therapy. Any therapeutic treatment of dementia can result in a change of the concentration of any of these substances and consequently, these concentrations may be used for monitoring or evaluating or determining the progress of the therapy. The disclosure pertaining to the method of diagnosing dementia equally applies to the method of monitoring the progress of a therapy for dementia.

In addition or alternatively, the invention is directed to a method for monitoring the progress of a therapy for dementia and optionally infer a prognosis comprising receiving a first sample from a subject at a first timepoint, measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the first sample, receiving a second sample from the subject at a second timepoint, measuring the concentration of NNN-trimethyl-5-aminovalerate and/or precursors of NNN-trimethyl-5-aminovalerate including but not limited to 5-aminovalerate and N^{ε}-trimethyllysine (N(6),N(6),N(6)-trimethyl-L-lysine) and/or metabolites of NNN-trimethyl-5-aminovalerate in the second sample, determining the progress of the therapy based on a comparison of the concentrations measured before. The disclosure pertaining to the method of diagnosing dementia equally applies to the method of monitoring the progress of a therapy for dementia.

### Experimental Procedures:

The above disclosed experiments have been performed with the following materials and methods. It is to be understood that the disclosure is not limited to these materials and methods and the same results as discussed can also be achieved using different materials and different methods.

Mice: Specific pathogen-free (SPF) C57BL/6 mice were purchased from Taconic Farms. Male and female mice of two age groups were used for the experiments; young (2 months old) mice and old mice (20-24 months old). Mice were group housed up to five per cage with 12 hr light/dark cycle with lights on at 6:00 a.m. Food and water were available ad libitum.

**Metabolomic analyses:** Blood serum and hippocampal brain tissue was removed from young and old mice and snap-frozen in liquid nitrogen after determining the weight. Sample analysis was conducted by Metabolon, Inc. using a proprietary series of organic and aqueous extractions to remove the protein content while allowing maximum recovery of small molecules. The extract was divided into two parts: one for analysis by LC and the other for analysis by GC. TurboVap® (Zymark) was used to remove the organic solvent content. Each sample was then frozen and dried under vacuum. The exact methodology can be provided by Metabolon, Inc. as their standard protocol for untargeted mass spectrometry metabolomics.

**Faecal microbiota transplantation (FMT):** For microbiota depletion studies, SPF mice were treated with 1 mg/ml of ampicillin (Auromedics), neomycin sulfate (Fisher), streptomycin (Sigma) and 0.5 mg/ml of vancomycin (Sagent) in the drinking water for 4-5 weeks (Khosravi et al., 2014). These mice were recolonized via FMT, which was achieved by oral gavage of a faecal slurry. Recipient mice had the food removed from the cage for 2 h prior to FMT. The faecal slurry was obtained by pooling faecal pellets from 8-14 donor mice. The pellets were weighed and resuspended by vortexing for 1 min in 1 mL PBS per 300 mg of faeces. After pelleting larger particles by centrifugation at 500 × g for 5 min, the supernatant was collected for FMT. Each recipient mouse received 150 µl of faecal slurry by oral gavage. The remaining slurry was stored at -80 °C for 16S rRNA sequencing. Following FMT, the cages of recipient mice were replenished with dirty bedding and fresh faecal pellets from donor mice once and twice a week, respectively. Faecal pellets for 16S rRNA sequencing were collected the day before FMT and at the end of the experiment and were stored at -80 °C for DNA extraction.

**Mouse injections:** NNN-trimethyl-5-aminovalerate was dissolved in 0.9% NaCl and administered i.p. at a dose of 200 mg/kg in a volume of 10 ml/kg. As control the vehicle NaCl was injected.

**Behavioral Testing:** Mice were tested 40 min after drug- or vehicle injection or 4 weeks after FMT. We first performed the training run for the Novel-object recognition test, followed by the T maze test and then 6 h after the training session mice were tested in the Novel object recognition test.

T maze: T Maze Spontaneous Alternation is a behavioral test for measuring exploratory behavior in animals, especially rodent models for CNS disorders. This test is based on the willingness of rodents to explore a new environment, i.e. they prefer to visit a new arm of the maze rather than a familiar arm. Many parts of the brain-including the hippocampus-are involved in this task. Subjects are first placed in the start arm of the T Maze. Upon leaving the start arm, mice choose between entering either the left or the right goal arm. With repeated trials (total of 15), the animals show less of a tendency to enter a previously visited arm. The percentage of alternation (number of turns in each goal arm) and total trial duration are recorded. This test is used to quantify cognitive deficits in transgenic strains of mice and evaluate novel chemical entities for their effects on cognition.

Novel Object Recognition: The Novel Object Recognition (NOR) task is used to evaluate cognition, particularly recognition memory, in rodent models of CNS disorders. This test is based on the spontaneous tendency of rodents to spend more time exploring a novel object than a familiar one. The choice to explore the novel object reflects the use of learning and recognition memory. The Novel Object Recognition task is conducted in an open field arena with two different kinds of objects. Both objects are generally consistent in height and volume but are different in shape and appearance. The animals are exposed to the familiar arena with two identical objects placed at an equal distance. After the animals have investigated the objects for a total of 20 s the mice are put in their home-cages. The second phase of testing is performed 6 hours after the training run. The mice are allowed to explore the open field in the presence of the familiar object and a novel object to test long-term recognition memory. The time spent exploring each object within 10 min and the discrimination index percentage is recorded. This test is useful for assessing impaired cognitive ability in transgenic strains of mice and evaluating novel chemical entities for their effect on cognition.

### Electrophysiology:

Hippocampal slice preparation: Mice were anesthetized and decapitated, after which the brain was quickly removed and placed into oxygenated, ice-cold, high-sucrose artificial cerebrospinal fluid (hsACSF), containing (in mM): 150 sucrose, 50 NaCl, 25 NaHCO3, 10 dextrose, 2.5 KCl, 1 NaH2PO4·H2O, 0.5 CaCl2, and 7 MgCl2. After 2 min in hsACSF, the brain was blocked, glued to the stage of a Vibratome (VTS1000; Leica, Bannockburn, IL), and cut into 300-µm horizontal brain slices containing hippocampus. Slices were then placed in a chamber containing ACSF (in mM: 124 NaCl, 3 KCl, 1.25 NaH2PO4·H2O, 2 MgSO4·7H2O, 26 NaHCO3, 10 dextrose, and 2 CaCl2). After a 40-min incubation period at 35°C, slices were maintained at room temperature (6-8 h).

Recordings: For recording, slices were placed in a submerged recording chamber (Warner Instruments, Hamden, CT) with oxygenated ACSF heated to 32°C and flowing at 2-4 ml/min. Interneurons were identified in area CA1 under infrared differential interference contrast (IR-DIC) optics. A micropipette puller (Sutter Instrument, Novato, CA) was used to pull patch pipettes of between 3 and 7 MΩ from 1.5-mm-OD borosilicate glass (World Precision Instruments, Sarasota, FL). Data were obtained using an Axopatch 1D amplifier (Axon Instruments, Foster City, CA); data were digitized at 10 kHz and recorded using a Digidata 1320A and pClamp 8.2 software (Molecular Devices, Sunnyvale, CA). For all experiments, the internal recording solution (pH 7.20-7.25; 285-295 mOsm) contained (in mM): 40 K-gluconate, 100 KCl, 2 NaCl, 10 HEPES, 4 EGTA, 4 MgATP, 0.3 Na2GTP, and 1.25 QX-314. For all experiments, mice were between P50 and P60. After obtaining a stable voltage-clamp recording from a CA1 interneuron, the recording was switched to current-clamp mode and injected steps of hyperpolarizing and depolarizing current were used to determine the class of cell being recorded using published firing patterns and action potential (AP) properties (Butt et al., 2005; Flames and Marin, 2005). The recording was subsequently switched back to voltage-clamp mode (holding potential, -60 mV) for recording of sIPSCs. Spontaneous IPSCs were pharmacologically isolated by perfusing the slice with ACSF containing 20 µM 6,7-dinitroquinoxaline-2,3-dione (DNQX, an α-amino-3-hydroxy-5-methyl-4-isoxazolepropionic acid [AMPA] receptor blocker) and 50 µM d-2-amino-5-phosphonovaleric acid (d-APV, an NMDA receptor blocker; Sigma). All recordings were obtained at 32°C. Voltage-clamp recordings were low-pass filtered at 1 kHz and band-pass filtered at 60 Hz (Hum Bug; AutoMate Scientific, Berkeley, CA). Whole cell access resistance and holding current were continuously monitored to confirm that recordings were stable. All recordings were first performed under control conditions and then in the presence of 10 µM NNN-trimethyl-5-aminovalerate in the bath solution.

In vivo electrophysiology: Local field potential were recorded with a wireless amplifier system (W4, Multichannel Systems) 2-21 days after surgery at a sampling rate of 2 kHz or with a wired recording system (RHD2000, Intan Technologies, sampling rate 10 kHz) on freely moving mice. Single-unit activity was recorded at 20-30 kHz using a 32-channel amplifier system (RHD2000, Intan Technologies). After the last recording session, the animals were deeply anaesthetized with an intraperitoneal injection of urethane (2 g/kg). To identify recording locations, electrolytic lesions were made by briefly (∼1 s) applying 10-20V to each electrode. The animals were intracardially perfused with phosphate-buffered saline (∼1min) followed by 4% paraformaldehyde (∼10min). The brains were sectioned (slice thickness 100-200µm) and inspected with a light microscope. A subset of brains were stained with cresyl violet or 4',6'-diamidino-2-phenylindole. Only recording areas located in the prelimbic or infralimbic cortex of the mPFC were accepted for analysis.

Data analysis. LFP data were analyzed with build-in and custom-made routines running in the Python 2.7 Spyder IDE. The raw LFP data of each mouse were converted to a z-score by subtracting the mean LFP and dividing by the standard deviation of the signal.

### Data and Statistical Analysis.

Data are expressed as mean ± SEM. Statistical analysis was performed with Prism 7.0 software (GraphPad Software). Means between two groups were compared with two-tailed, unpaired Student's t test. Comparisons of means from multiple groups with each other or against one control group were analyzed with 1-way ANOVA and Bonferroni post hoc tests. All electrophysiology and behavior experiments conducted were done in a randomized and blinded fashion.

### Scientific Literature:

Ahluwalia, V., Betrapally, N.S., Hylemon, P.B., White, M.B., Gillevet, P.M., Unser, A.B., Fagan, A., Daita, K., Heuman, D.M., Zhou, H., et al. (2016). Impaired Gut-Liver-Brain Axis in Patients with Cirrhosis. Sci Rep 6, 26800.

Bercik, P., Denou, E., Collins, J., Jackson, W., Lu, J., Jury, J., Deng, Y., Blennerhassett, P., Macri, J., McCoy, K.D., et al. (2011). The intestinal microbiota affect central levels of brain-derived neurotropic factor and behavior in mice. Gastroenterology 141, 599-609, 609 e591-593.

Butt, S.J., Fuccillo, M., Nery, S., Noctor, S., Kriegstein, A., Corbin, J.G., and Fishell, G. (2005). The temporal and spatial origins of cortical interneurons predict their physiological subtype. Neuron 48, 591-604.

Buzsaki, G., and Draguhn, A. (2004). Neuronal oscillations in cortical networks. Science 304, 1926-1929.

Buzsaki, G., and Wang, X.J. (2012). Mechanisms of gamma oscillations. Annu Rev Neurosci 35, 203-225.

Caspani, G., Kennedy, S., Foster, J.A., and Swann, J. (2019). Gut microbial metabolites in depression: understanding the biochemical mechanisms. Microb Cell 6, 454-481.

Dai, Z.L., Wu, G., and Zhu, W.Y. (2011). Amino acid metabolism in intestinal bacteria: links between gut ecology and host health. Front Biosci (Landmark Ed) 16, 1768-1786.

Dam, S.A., Mostert, J.C., Szopinska-Tokov, J.W., Bloemendaal, M., Amato, M., and Arias-Vasquez, A. (2019). The Role of the Gut-Brain Axis in Attention-Deficit/Hyperactivity Disorder. Gastroenterol Clin North Am 48, 407-431.

Flames, N., and Marin, O. (2005). Developmental mechanisms underlying the generation of cortical interneuron diversity. Neuron 46, 377-381.

Fries, P. (2015). Rhythms for Cognition: Communication through Coherence. Neuron 88, 220-235.

Grenham, S., Clarke, G., Cryan, J.F., and Dinan, T.G. (2011). Brain-gut-microbe communication in health and disease. Front Physiol 2, 94.

Khosravi, A., Yanez, A., Price, J.G., Chow, A., Merad, M., Goodridge, H.S., and Mazmanian, S.K. (2014). Gut microbiota promote hematopoiesis to control bacterial infection. Cell Host Microbe 15, 374-381.

Klausberger, T., and Somogyi, P. (2008). Neuronal diversity and temporal dynamics: the unity of hippocampal circuit operations. Science 321, 53-57.

Knorr, S., Sinn, M., Galetskiy, D., Williams, R.M., Wang, C., Muller, N., Mayans, O., Schleheck, D., and Hartig, J.S. (2018). Widespread bacterial lysine degradation proceeding via glutarate and L-2-hydroxyglutarate. Nat Commun 9, 5071.

Maclin, J.M.A., Wang, T., and Xiao, S. (2019). Biomarkers for the diagnosis of Alzheimer's disease, dementia Lewy body, frontotemporal dementia and vascular dementia. Gen Psychiatr 32, e100054.

Mizoguchi, K., Shoji, H., Tanaka, Y., Maruyama, W., and Tabira, T. (2009). Age-related spatial working memory impairment is caused by prefrontal cortical dopaminergic dysfunction in rats. Neuroscience 162, 1192-1201.

Nyberg, L., Lovden, M., Riklund, K., Lindenberger, U., and Backman, L. (2012). Memory aging and brain maintenance. Trends Cogn Sci 16, 292-305.

Rhee, S.H., Pothoulakis, C., and Mayer, E.A. (2009). Principles and clinical implications of the brain-gut-enteric microbiota axis. Nat Rev Gastroenterol Hepatol 6, 306-314.

Saji, N., Niida, S., Murotani, K., Hisada, T., Tsuduki, T., Sugimoto, T., Kimura, A., Toba, K., and Sakurai, T. (2019). Analysis of the relationship between the gut microbiome and dementia: a cross-sectional study conducted in Japan. Sci Rep 9, 1008.

Schneider, B.L., Ruback, S., Kiupakis, A.K., Kasbarian, H., Pybus, C., and Reitzer, L. (2002). The Escherichia coli gabDTPC operon: specific gamma-aminobutyrate catabolism and nonspecific induction. J Bacteriol 184, 6976-6986.

Shen, T.C., Albenberg, L., Bittinger, K., Chehoud, C., Chen, Y.Y., Judge, C.A., Chau, L., Ni, J., Sheng, M., Lin, A., et al. (2015). Engineering the gut microbiota to treat hyperammonemia. J Clin Invest 125, 2841-2850.

Sun, J., Xu, J., Ling, Y., Wang, F., Gong, T., Yang, C., Ye, S., Ye, K., Wei, D., Song, Z., et al. (2019). Fecal microbiota transplantation alleviated Alzheimer's disease-like pathogenesis in APP/PS1 transgenic mice. Transl Psychiatry 9, 189.

Yang, Z., and Lu, C.D. (2007). Functional genomics enables identification of genes of the arginine transaminase pathway in Pseudomonas aeruginosa. J Bacteriol 189, 3945-3953.

The above described invention may also be described with the following embodiments:
1. A method for diagnosing the probability of a subject developing or having dementia, the method comprising:
   a) receiving a sample from a subject;
   b) measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the sample;
   c) determining the probability of the subject developing or having dementia based on the concentration measured in step b.
2. The method of embodiment 1, wherein the sample is selected from one of a saliva sample, a urine sample, a blood sample, a serum sample, a sample of brain liquor, a sample of ventricular fluid, a sample of spinal fluid, a brain tissue sample, a microbial sample, a faecal sample or a stool sample.
3. The method of any of embodiments 1 and 2, wherein the subject is human.
4. The method of any of embodiments 1 to 3, wherein a concentration of NNN-trimethyl-5-aminovalerate between 0.005 and 0,050 µM/g creatinine in urine is indicative for the subject developing or having dementia.
5. The method of any of embodiments 1 to 3, wherein the precursor of NNN-trimethyl-5-aminovalerate is selected from one of 5-aminovalerate or N^{ε}-trimethyllysine (N(6),N(6),N(6)-trimethyl-L-lysine).
6. The method of embodiment 5, wherein a concentration of 5-aminovalerate between 0.005 and 0,050 µM/g creatinine in urine or a concentration of N^{ε}-trimethyllysine between 4 and 8 µM/g creatinine in urine is indicative for the subject developing or having dementia.
7. The method of any of embodiments 1 to 3, wherein the metabolite of NNN-trimethyl-5-aminovalerate is selected from one of glutaric acid and 5-(galactosyl hydroxy)-L-lysine.
8. The method of embodiment 7, wherein a concentration of glutaric acid between 6-24 µM/g creatinine in urine, between 10-20 µM in serum, between 10-40 mM in cerebrospinal fluid, or a concentration of 5-(galactosyl hydroxy)-L-lysine between 1.4 and 2 µM/g creatinine in urine and between 0.1 and 0.3 µM in serum is indicative for the subject developing or having dementia.
9. The method of any of the preceding embodiments, wherein the concentration is determined by comparison to internal standards or by external comparison to metabolite standards.
10. The method of any of the preceding embodiments, wherein the concentration is determined using any of the following methods: liquid chromatography-mass spectrometry (LC-MS), nuclear magnetic resonance (NMR) or immunoassays.
11. The method of any of the preceding embodiments, wherein step c) comprises comparing the concentration of step b) with control data, in particular control data from one or more healthy individuals of the same age, same sex, same ethnicity, and/or same geographical location.
12. The method of any of the preceding embodiments, wherein the dementia is selected from one of the following: Alzheimer's disease, Parkinson's disease, Huntington disease, frontotemporal dementia, amyotrophic lateral sclerosis, multiple sclerosis, glaucoma, myotonic dystrophy, progressive supranuclear palsy, spinal muscular atrophy, multi-system atrophy, ataxias, vascular dementia, or other dementias.
13. A method for diagnosing the probability of a subject developing or having dementia, the method comprising:
   a) receiving a first sample from a subject at a first timepoint;
   b) measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the first sample;
   c) receiving a second sample from the subject at a second timepoint;
   d) measuring the concentration of NNN-trimethyl-5-aminovalerate and/or precursors of NNN-trimethyl-5-aminovalerate including but not limited to 5-aminovalerate and N^{ε}-trimethyllysine (N(6),N(6),N(6)-trimethyl-L-lysine) and/or metabolites of NNN-trimethyl-5-aminovalerate in the second sample;
   e) determining the probability of the subject developing or having dementia based on a comparison of the concentrations measured in steps b and d.
14. The method of embodiment 13, wherein one or more of the samples are selected from a blood sample, a serum sample, a sample of brain liquor, a sample of ventricular fluid, a sample of spinal fluid, a brain tissue sample, a microbial sample, a faecal sample, a saliva sample, a urine sample or a stool sample.
15. The method of any of embodiments 13 and 14, wherein the subject is human.
16. The method of any of embodiments 13 to 15, wherein the first and second time points are separated by about 3-6 months.
17. The method of any of embodiments 13 to 16, wherein the second timepoint is 12 to 24 weeks after the first timepoint.
18. The method of any of embodiments 13 to 17, wherein the second timepoint is 3 to 6 months after the first timepoint.
19. The method of any of embodiments 13 to 18 further comprising
   f) receiving a third sample from a subject at a third timepoint;
   g) measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the third sample;
   h) determining the probability of the subject developing or having dementia based on a comparison of the concentrations measured in steps b, d and g.
20. The method of embodiment 19, wherein the second and third time point are separated by 6-12 months.
21. The method of any of embodiment 13 to 20, wherein further samples are received at further timepoints and wherein the probability of the subject developing or having dementia is based on a comparison of the concentrations measured in the samples.
22. The method of any of embodiments 13 to 21, wherein the dementia is an age-related or age-unrelated dementia.
23. The method of any of embodiments 13 to 22, wherein the dementia is selected from one of the following: Alzheimer's disease, Parkinson's disease, Huntington disease, frontotemporal dementia, amyotrophic lateral sclerosis, multiple sclerosis, glaucoma, myotonic dystrophy, progressive supranuclear palsy, spinal muscular atrophy, multi-system atrophy, ataxias, vascular dementia, or other dementias.
24. The method of any of embodiments 13 to 23, wherein the concentration is measured in vivo with a sensor or with imaging related methods.
25. A method for diagnosing the probability of a subject developing or having dementia, the method comprising:
   a) receiving a sample from a subject;
   b) determining the abundance of any of Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the sample;
   c) determining the probability of the subject developing or having dementia based on the abundance measured in step b.
26. The method of embodiment 25, wherein the sample is selected from one or more of a microbial sample, a gut flora sample, an intestinal sample, a faecal sample and/or a stool sample.
27. The method of any of embodiments 25 and 26, wherein the subject is human.
28. The method of any of embodiments 25 to 27, wherein the Corynebacterium is selected from one or more of Corynebacterium glutamicum, Corynebacterium jeikeium, Corynebacterium urealyticum and Corynebacterium efficiens.
29. The method of any of embodiments 25 to 28, wherein the Oscillibacter is selected from Oscillibacter valerigens and Oscillibacter sp., strain KLE 1745.
30. The method of any of embodiments 25 to 29, wherein the composition of the microbiota in the sample is determined.
31. The method of any of embodiments 25 to 30, wherein a gut metagenome is determined
32. The method of any of embodiments 25 to 31, wherein the method comprises comparing the abundance of the bacteria or the composition of the microbiota or the gut metagenome of the sample of the subject with a control.
33. The method of embodiment 32, wherein the control is based on data from one or more healthy individuals.
34. The method of embodiment 33, wherein the control is determined from one or more healthy individuals of the same age, same sex, same ethnicity, and/or same geographical location.
35. The method of any of embodiments 25 to 34, wherein the sample is analyzed using any of the following methods: sequence-based techniques, genotyping assays, qPCR, RT-qPCR, clone library of full-length 16S rRNA gene sequences, DGGE, T-RFLP, ARISA, microarrays, DNA hybridization methods.
36. The method of any of embodiments 25 to 35, wherein the abundance of bacteria is measured using a cell culture assay including at least one of culture in suspension or on a plate, staining, microscopy, flow cytometrical methods such as FACS, optical density measurements.
37. The method of any of embodiments 25 to 36, wherein the dementia is an age-related or age-unrelated dementia.
38. The method of any of embodiments 25 to 37, wherein the dementia is selected from one of the following: Alzheimer's disease, Parkinson's disease, Huntington disease, frontotemporal dementia, amyotrophic lateral sclerosis, multiple sclerosis, glaucoma, myotonic dystrophy, progressive supranuclear palsy, spinal muscular atrophy, multi-system atrophy, ataxias, vascular dementia, or other dementias.
39. A method for diagnosing the probability of a subject developing or having dementia, the method comprising:
   a) identifying parvalbumin-positive interneurons in a sample;
   b) measuring the frequency of spontaneous IPSCs in the sample;
   c) determining the probability of the subject developing or having dementia based on the frequency measured in step b.
40. The method of embodiment 39, wherein the sample is selected from a brain, an acute brain slice, cultured brain tissue, a culture of neurons, or a culture of parvalbumin-positive interneurons.
41. The method of any of embodiments 39 and 40, wherein the subject is human.
42. The method of any of embodiments 39 to 41, wherein the spontaneous IPSCs are measured using electrophysiological methods and/or calcium imaging.
43. The method of any of embodiments 39 to 42, wherein oscillations of PV-positive GABA neurons are detected by electroencephalography (EEG) and magnetoencephalography (MEG).
44. A method for screening for a drug candidate, the method comprising:
   a) providing a sample including one or more of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine;
   b) subjecting the sample to a test agent;
   c) measuring the effect of the test agent on the sample;
   d) determining based on the effect of the test agent on the sample the suitability of the test agent as a drug candidate.
45. A method for treating a subject developing or having dementia or being at risk of developing dementia, the method comprising:
   administering to the subject an agent, which reduces the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the subject.
46. The method of embodiment 45, wherein the agent or the combination of agents reduces the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in a saliva sample, a urine sample, a blood sample, a serum sample, a sample of brain liquor, a sample of ventricular fluid, a sample of spinal fluid, a brain tissue sample, a microbial sample, a faecal sample or a stool sample.
47. The method of embodiment 45 or 46, wherein the method further comprises
   a) receiving a sample from the subject;
   b) measuring the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the sample;
   c) determining the progress and/or prognosis of the treatment based on the concentration measured in step b,
   wherein the method optionally comprises adjusting the treatment based on the progress determined in step c).
48. The method of embodiments 45 or 46, wherein the method further comprises
   a) receiving a first sample from the subject at a first timepoint;
   b) measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the first sample;
   c) receiving a second sample from the subject at a second timepoint;
   d) measuring the concentration of NNN-trimethyl-5-aminovalerate and/or precursors of NNN-trimethyl-5-aminovalerate including but not limited to 5-aminovalerate and N^{ε}-trimethyllysine (N(6),N(6),N(6)-trimethyl-L-lysine) and/or metabolites of NNN-trimethyl-5-aminovalerate in the second sample;
   e) determining the progress and/or prognosis of the therapy based on a comparison of the concentrations measured in steps b and d,
   wherein the method optionally comprises adjusting the treatment based on the progress determined in step e).
49. The method of embodiment 48, wherein the first and second time points are separated by about 3-6 months.
50. The method of any of embodiments 47 to 49, wherein one or more of the samples are selected from a blood sample, a serum sample, a sample of brain liquor, a sample of ventricular fluid, a sample of spinal fluid, a brain tissue sample, a microbial sample, a faecal sample, a saliva sample, a urine sample or a stool sample.
51. A method for treating a subject developing or having dementia, the method comprising:
   administering to the subject an agent which reduces or eradicates any of the bacteria of the phylum Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the gut flora.
52. The method of embodiment 51, wherein the agent comprises an antimicrobial agent or a vaccine or a topical probiotic intervention, or another bacterium which directly or indirectly has an influence on the abundance of the bacteria of embodiment 51.
53. A method for identifying a patient group being suitable for a treatment of dementia, the method comprising:
   a) receiving a sample from a subject;
   b) measuring the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the sample;
   c) determining the probability of the subject being responsive to a treatment based on the concentration measured in step b.
54. The method of embodiment 53, wherein the treatment comprises administering to the subject an agent or the combination of agents, which reduces the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the subject.
55. A method for identifying a patient group being suitable for a treatment of dementia, the method comprising:
   a) receiving a sample from a subject;
   b) determining the abundance of any of Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the sample;
   c) determining the probability of the subject being responsive to a treatment based on the abundance measured in step b.
56. The method of embodiment 55, wherein the treatment comprises administering to the subject an agent which reduces or eradicates any of the bacteria of the phylum Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the gut flora.
57. The method of embodiment 56, wherein the agent comprises an antimicrobial agent or a vaccine or a topical probiotic intervention, or another bacterium which directly or indirectly has an influence on the abundance of the bacteria of embodiment 56.
58. A method for monitoring the progress of a therapy for dementia comprising:
   a) receiving a sample from a subject;
   b) measuring the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the sample;
   c) determining the progress and/or prognosis of the therapy based on the concentration measured in step b.
59. The method of embodiment 58, wherein the therapy is a therapy which influences the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the subject.
60. The method of embodiment 58 or 59, wherein the sample is selected from one of a saliva sample, a urine sample, a blood sample, a serum sample, a sample of brain liquor, a sample of ventricular fluid, a sample of spinal fluid, a brain tissue sample, a microbial sample, a faecal sample or a stool sample.
61. The method of any of embodiments 58 to 60, wherein the subject is human.
62. The method of any of embodiments 58 to 61, wherein a concentration of NNN-trimethyl-5-aminovalerate between 0.005 and 0,050 µM/g creatinine in urine is indicative for the subject developing or having dementia.
63. The method of any of embodiments 58 to 62, wherein the precursor of NNN-trimethyl-5-aminovalerate is selected from one of 5-aminovalerate or N^{ε}-trimethyllysine (N(6),N(6),N(6)-trimethyl-L-lysine).
64. The method of embodiment 63, wherein a concentration of 5-aminovalerate between 0.005 and 0,050 µM/g creatinine in urine or a concentration of N^{ε}-trimethyllysine between 4 and 8 µM/g creatinine in urine is indicative for the subject developing or having dementia.
65. The method of any of embodiments 58 to 61, wherein the metabolite of NNN-trimethyl-5-aminovalerate is selected from one of glutaric acid and 5-(galactosyl hydroxy)-L-lysine.
66. The method of embodiment 65, wherein a concentration of glutaric acid between 6-24 µM/g creatinine in urine, between 10-20 µM in serum, between 10-40 mM in cerebrospinal fluid, or a concentration of 5-(galactosyl hydroxy)-L-lysine between 1.4 and 2 µM/g creatinine in urine and between 0.1 and 0.3 µM in serum is indicative for the subject developing or having dementia.
67. The method of any of embodiments 58 to 66, wherein the concentration is determined by comparison to internal standards or by external comparison to metabolite standards.
68. The method of any of embodiments 58 to 67, wherein the concentration is determined using any of the following methods: liquid chromatography-mass spectrometry (LC-MS), nuclear magnetic resonance (NMR) or immunoassays.
69. The method of any of embodiments 58 to 68, wherein step c) comprises comparing the concentration of step b) with control data, in particular control data from one or more healthy individuals of the same age, same sex, same ethnicity, and/or same geographical location.
70. The method of any of embodiments 58 to 69, wherein the dementia is selected from one of the following: Alzheimer's disease, Parkinson's disease, Huntington disease, frontotemporal dementia, amyotrophic lateral sclerosis, multiple sclerosis, glaucoma, myotonic dystrophy, progressive supranuclear palsy, spinal muscular atrophy, multi-system atrophy, ataxias, vascular dementia, or other dementias.
71. A method for monitoring the progress and/or prognosis of a therapy for dementia comprising:
   a) receiving a first sample from a subject at a first timepoint;
   b) measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the first sample;
   c) receiving a second sample from the subject at a second timepoint;
   d) measuring the concentration of NNN-trimethyl-5-aminovalerate and/or precursors of NNN-trimethyl-5-aminovalerate including but not limited to 5-aminovalerate and N^{ε}-trimethyllysine (N(6),N(6),N(6)-trimethyl-L-lysine) and/or metabolites of NNN-trimethyl-5-aminovalerate in the second sample;
   e) determining the progress of the therapy based on a comparison of the concentrations measured in steps b and d.
72. The method of embodiment 71, wherein one or more of the samples are selected from a blood sample, a serum sample, a sample of brain liquor, a sample of ventricular fluid, a sample of spinal fluid, a brain tissue sample, a microbial sample, a faecal sample, a saliva sample, a urine sample or a stool sample.
73. The method of any of embodiments 71 and 72, wherein the subject is human.
74. The method of any of embodiments 71 to 73, wherein the first and second time points are separated by about 3-6 months.
75. The method of any of embodiments 71 to 74, wherein the second timepoint is 12 to 24 weeks after the first timepoint.
76. The method of any of embodiments 71 to 75, wherein the second timepoint is 3 to 6 months after the first timepoint.
77. The method of any of embodiments 71 to 76 further comprising
   f) receiving a third sample from a subject at a third timepoint;
   g) measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the third sample;
   h) determining the progress of the therapy based on a comparison of the concentrations measured in steps b, d and g.
78. The method of embodiment 77, wherein the second and third time point are separated by 6-12 months.
79. The method of any of embodiment 71 to 78, wherein further samples are received at further timepoints and wherein the probability of the subject developing or having dementia is based on a comparison of the concentrations measured in the samples.
80. The method of any of embodiments 71 to 79, wherein the dementia is an age-related or age-unrelated dementia.
81. The method of any of embodiments 71 to 80, wherein the dementia is selected from one of the following: Alzheimer's disease, Parkinson's disease, Huntington disease, frontotemporal dementia, amyotrophic lateral sclerosis, multiple sclerosis, glaucoma, myotonic dystrophy, progressive supranuclear palsy, spinal muscular atrophy, multi-system atrophy, ataxias, vascular dementia, or other dementias.
82. The method of any of embodiments 71 to 81, wherein the concentration is measured in vivo with a sensor or with imaging related methods.

## Claims

1. A method for diagnosing the probability of a subject developing or having dementia, the method comprising:
a) receiving a sample from a subject;
b) measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the sample;
c) determining the probability of the subject developing or having dementia based on the concentration measured in step b.

2. The method of claim 1, wherein the sample is selected from one of a saliva sample, a urine sample, a blood sample, a serum sample, a sample of brain liquor, a sample of ventricular fluid, a sample of spinal fluid, a brain tissue sample, a microbial sample, a faecal sample or a stool sample.

3. The method of any of claims 1 to 2, wherein a concentration of NNN-trimethyl-5-aminovalerate between 0.005 and 0,050 µM/g creatinine in urine is indicative for the subject developing or having dementia.

4. The method of any of claims 1 to 2, wherein the precursor of NNN-trimethyl-5-aminovalerate is selected from one of 5-aminovalerate or N^{ε}-trimethyllysine (N(6),N(6),N(6)-trimethyl-L-lysine).

5. The method of any of the preceding claims, wherein the concentration is determined by comparison to internal standards or by external comparison to metabolite standards.

6. The method of any of the preceding claims, wherein step c) comprises comparing the concentration of step b) with control data, in particular control data from one or more healthy individuals of the same age, same sex, same ethnicity, and/or same geographical location.

7. The method of any of the preceding claims, wherein the dementia is selected from one of the following: Alzheimer's disease, Parkinson's disease, Huntington disease, frontotemporal dementia, amyotrophic lateral sclerosis, multiple sclerosis, glaucoma, myotonic dystrophy, progressive supranuclear palsy, spinal muscular atrophy, multi-system atrophy, ataxias, vascular dementia, or other dementias.

8. A method for diagnosing the probability of a subject developing or having dementia, the method comprising:
a) receiving a first sample from a subject at a first timepoint;
b) measuring the concentration of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the first sample;
c) receiving a second sample from the subject at a second timepoint;
d) measuring the concentration of NNN-trimethyl-5-aminovalerate and/or precursors of NNN-trimethyl-5-aminovalerate including but not limited to 5-aminovalerate and N^{ε}-trimethyllysine (N(6),N(6),N(6)-trimethyl-L-lysine) and/or metabolites of NNN-trimethyl-5-aminovalerate in the second sample;
e) determining the probability of the subject developing or having dementia based on a comparison of the concentrations measured in steps b and d.

9. The method of claim 8, wherein the first and second time points are separated by about 3-6 months.

10. A method for diagnosing the probability of a subject developing or having dementia, the method comprising:
a) receiving a sample from a subject;
b) determining the abundance of any of Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the sample;
c) determining the probability of the subject developing or having dementia based on the abundance measured in step b.

11. The method of claim 10, wherein the sample is selected from one or more of a microbial sample, a gut flora sample, an intestinal sample, a faecal sample and/or a stool sample.

12. A method for diagnosing the probability of a subject developing or having dementia, the method comprising:
a) receiving a brain sample from a human being;
b) identifying parvalbumin-positive interneurons in the brain sample;
c) measuring the frequency of spontaneous IPSCs in the brain sample;
d) determining the probability of the subject developing or having dementia based on the frequency measured in step c.

13. A method for screening for a drug candidate, the method comprising:
a) providing a sample including one or more of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine;
b) subjecting the sample to a test agent;
c) measuring the effect of the test agent on the sample;
d) determining based on the effect of the test agent on the sample the suitability of the test agent as a drug candidate.

14. A method for identifying a patient group being suitable for a treatment of dementia, the method comprising:
a) receiving a sample from a subject;
b) measuring the concentration of any of NNN-trimethyl-5-aminovalerate and/or precursors of NNN-trimethyl-5-aminovalerate including but not limited to 5-aminovalerate and Nε-trimethyllysine (N(6),N(6),N(6)-trimethyl-L-lysine) and/or metabolites of NNN-trimethyl-5-aminovalerate including but not limited to glutaric acid and 5-(galactosyl hydroxy)-L-lysine in the sample;
c) determining the probability of the subject being responsive to a treatment based on the concentration measured in step b.

15. A method for identifying a patient group being suitable for a treatment of dementia, the method comprising:
a) receiving a sample from a subject;
b) determining the abundance of any of Corynebacterium, Clostridium sporogenes, Clostridium sticklandii, Clostridium perfringens, Clostridium butyricum, Clostridium sphenoides, Clostridium glutamicum, Clostridium bifermentans, Clostridioides difficile, Oscillibacter, and Cloacibacillus evryensi in the sample;
c) determining the probability of the subject being responsive to a treatment based on the abundance measured in step b.
